# FASCICULE DE BREVET EUROPEEN

(11) **EP 1 963 436 B1**
(45) Date de publication et mention de la délivrance du brevet: **25.04.2018**
(21) Numéro de dépôt: 07731808.7
(22) Date de dépôt: 23.03.2007
(51) Int. Cl.: C09B 57/08, C09B 69/02, A61Q 5/08, A61K 8/49

(54) **COLORANTS PARTICULIERS DE TYPE NAPHTALIMIDE, COMPOSITION TINCTORIALE COMPRENANT AU MOINS UN TEL COLORANT, PROCEDE DE MISE EN OEUVRE ET UTILISATIONS**
SPEZIFISCHE NAPHTHALIMIDFARBSTOFFE, FARBSTOFFZUSAMMENSETZUNG MIT ZUMINDEST EINEM DERARTIGEN FARBSTOFF, IMPLEMENTATIONSVERFAHREN UND VERWENDUNG
SPECIFIC DYES OF NAPHTHALIMIDE TYPE, DYEING COMPOSITION COMPRISING AT LEAST ONE SUCH DYE, METHOD OF IMPLEMENTATION AND USES

(30) Priorité: 24.03.2006 FR 0602608
(43) Date de publication de la demande: 03.09.2008
(73) Titulaire: L'Oréal, 75008 Paris (FR)
(72) Inventeur: LUUKAS, Timo, F-91300 Massy (FR); GREAVES, Andrew, F-77144 Montevrain (FR); DAVID, Hervé, F-94210 La Varenne Saint Hilaire (FR)
(74) Mandataire: Casalonga, Axel
(86) Numéro de dépôt international: PCT/FR2007/050995
(87) Numéro de publication internationale: WO 2007/110530

(56) Documents cités:
- WO-A-93/18789
- WO-A-2005/074872
- WO-A-2005/075574
- CH-A- 520 677
- HE TIAN, TAO XU, YONGBO ZHAO AND KONGCHANG CHEN: "Two-path photo-induced electron transfer in naphthalimide-based model compound" J. CHEM. SOC., PERKIN TRANS. 2,, vol. 3, 1999, pages 545-549, XP002419034
- M. LANGLOIS, J. L. SOULIER, M. MATHÉ-ALLAINMAT, C. GALLAIS, B. BRÉMONT AND S. SHEN: "N-chloromethyl quinuclidinium derivatives: A new class of irreversible ligands for 5-HT3 receptors" BIOORGANIC AND MEDICINAL CHEMISTRY LETTERS, vol. 4, no. 7, 1994, pages 945-948, XP002419035
- DATABASE REGISTRY AMERICAN CHEMICAL SOCIETY (ACS); FILE REGISTRY, RN = 81494-75-9 16 novembre 1984 (1984-11-16), "Pyridinium, 1-[2-[6-(dimethylamino)-1,3-dioxo-1H-benz[ de]isoquinolin-2(3H)-yl]ethyl]- (9CI) (CA INDEX NAME)" XP002419036
- DATABASE WPI Week 200433 Derwent Publications Ltd., London, GB; AN 2004-351444 XP002419038 -& JP 2004 095492 A (DAINIPPON PRINTING CO LTD) 25 mars 2004 (2004-03-25)

## Description

La présente demande a pour objet des colorants de type naphtalimide, leur utilisation en tant que colorants directs pour la teinture des fibres kératiniques ainsi que les compositions tinctoriales comprenant de tels composés.

Il est connu de teindre les fibres kératiniques par une coloration directe. Le procédé classiquement utilisé en coloration directe consiste à appliquer sur les fibres kératiniques des colorants directs qui sont des molécules colorées et colorantes ayant une affinité pour les fibres, à laisser poser, puis à rincer les fibres.

Il est connu par exemple d'utiliser des colorants directs du type nitré benzénique, anthraquinonique, nitropyridinique, des colorants du type azoïque, xanthénique, acridinique, azinique ou des colorants triarylméthane, par exemple.

Les colorations qui en résultent sont des colorations particulièrement chromatiques qui sont cependant temporaires ou semi-permanentes. En effet, la nature des intéractions qui lient les colorants directs à la fibre kératinique, et leur désorption de la surface et/ou du coeur de la fibre font que la puissance tinctoriale et la tenue aux lavages ou à la transpiration des colorations peuvent être encore considérées insuffisantes. Certains colorants directs peuvent, en outre, être sensibles à la lumière du fait de la faible résistance du chromophore vis-à-vis des attaques photochimiques et conduire dans le temps à un affadissement de la coloration des cheveux.

Afin d'obtenir une coloration plus visible, il est connu de décolorer les fibres kératiniques. Cette décoloration des fibres est effectuée par application d'un agent oxydant. Cependant, les colorants directs peuvent être sensibles à l'action des agents oxydants ce qui les rend généralement difficilement utilisables dans ces conditions. Par ailleurs, l'utilisation d'un agent oxydant peut dégrader de façon substantielle les propriétés cosmétiques des fibres kératiniques.

Par ailleurs, des dérivés de type naphtalimide sont connus de l'état de la technique depuis les années 1960 notamment en tant qu'azurants optiques ou agents de blanchiment pour des produits textiles synthétiques tels que les produits textiles acryliques ou en acétate de cellulose. Parmi les documents illustrant cette utilisation, on peut citer les brevets US 3,625,947, US 4,508,900, US 4,595,756 et la demande de brevet FR-A-1 557 945 qui concerne des composés 4-alcoxynaphtalimides cationiques, la demande de brevet FR-A- 2010 444 qui concerne des composés 4,5-dialcoxynaphtalimides cationiques et la demande de brevet FR-A-2 162 181 qui concerne des colorants azoïques comprenant un groupe naphtalimide.

Par ailleurs, la demande de brevet WO 2005/075574 décrit des colorants directs de type naphtalénique, cationiques ou neutres, pour teindre des fibres keratiniques et la demande de brevet WO 2005/074872 décrit des colorants directs de type naphtalénique pour éclaircir des fibres kératiniques.

Dans le domaine de la coloration directe des fibres kératiniques, il existe un réel besoin de rechercher des colorants directs très chromatiques et résistants aux agents extérieurs tels que les intempéries, les lavages et la transpiration.

Il existe aussi un réel besoin de disposer de colorants qui permettent d'obtenir des couleurs chromatiques et qui sont stables en milieu oxydant. Il existe aussi un réel besoin de disposer de colorant direct permettant un éclaircissement des fibres kératiniques même foncées sans faire appel à un agent oxydant.

Ainsi, la présente invention a pour objet un colorant naphtalimide correspondant aux composés de formule générale (I) ainsi que les dimères des composés (I) correspondant aux formules générales (II), (III) et (IV) suivantes, ainsi que leurs sels d'addition avec un acide et leurs solvates : 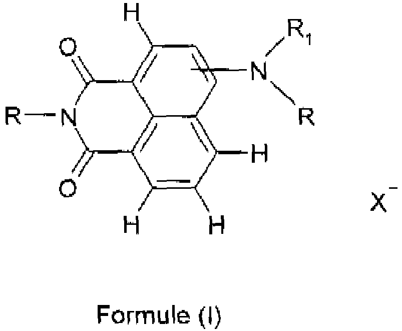 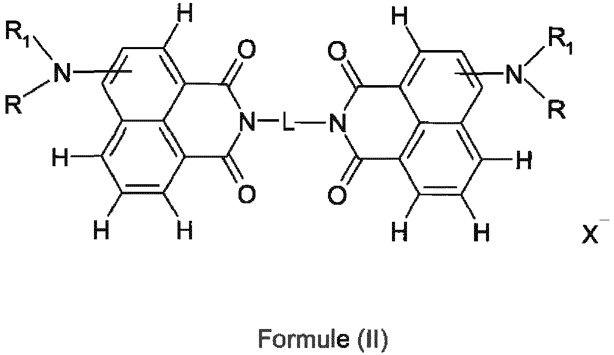 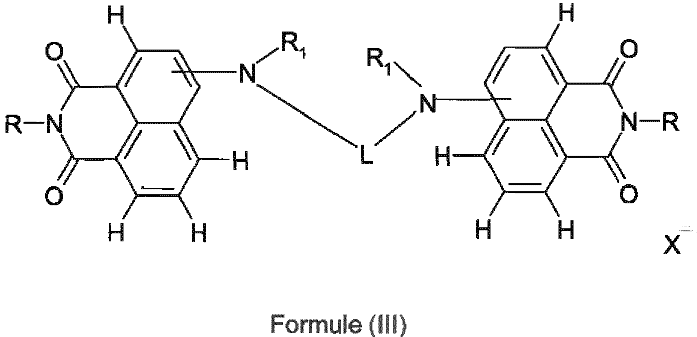 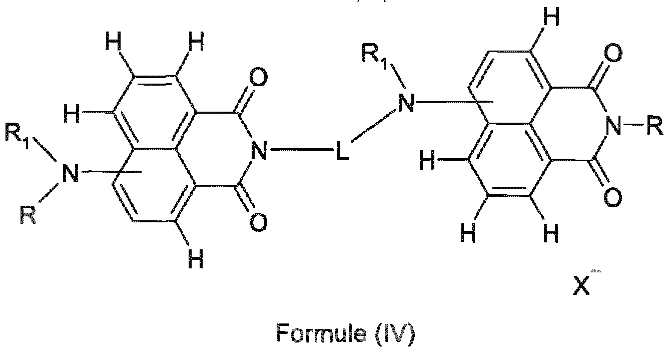 formules (I) à (IV) dans lesquelles :
- les radicaux R₁ pris séparément et indépendamment les uns des autres représentent un atome d'hydrogène ; un radical alkyle en C₁-C₄ éventuellement substitué par au moins un substituant choisi parmi les groupements :
   - hydroxyle,
   - alcoxy en C₁-C₄, (poly)hydroxyalcoxy en C₂-C₄,
   - amino, amino substitué par un ou deux groupements alkyle identiques ou différents en C₁-C₄ éventuellement porteurs d'au moins un groupement hydroxyle, alcoxy en C₁-C₂, lesdits radicaux alkyle pouvant former avec l'atome d'azote auquel ils sont rattachés, un hétérocycle comprenant de 5 ou 7 chaînons, saturé ou insaturé, éventuellement aromatique, éventuellement substitué, comprenant éventuellement au moins un autre hétéroatome différent ou non de l'azote.
   - Un radical alkylcarbonylamino (R'CO-NR-) dans lequel le radical R est un atome d'hydrogène, un radical alkyle en C₁-C₄ et le radical R' représente un radical alkyle en C₁-C₄, un radical phényle ;
   - Un radical alkylsulfonyle (R-SO₂-) dans lequel le radical R représente un radical alkyle en C₁-C₄.
   - Un radical alkylsulfinyle (R-SO-) dans lequel le radical R représente un radical alkyle en C₁-C₄.
   - Un radical alkylcarbonyle (R-CO-) dans lequel le radical R représente un radical alkyle en C₁-C₄,
- les radicaux R pris séparément et indépendamment les uns des autres représentent un atome d'hydrogène ; un radical aryle ou arylalkyle dont la partie aryle est éventuellement substituée ; un radical alkyle en C₁-C₈, de préférence en C₂-C₈, étant éventuellement substitué par au moins un substituant choisi parmi les groupements :
   - hydroxyle,
   - alcoxy en C₁-C₄, (poly)hydroxyalcoxy en C₂-C₄,
   - amino, amino substitué par un ou deux groupements alkyle identiques ou différents en C₁-C₄ éventuellement porteurs d'au moins un groupement hydroxyle, alcoxy en C₁-C₂, lesdits radicaux alkyle pouvant former avec l'atome d'azote auquel ils sont rattachés, un hétérocycle comprenant de 5 ou 7 chaînons, saturé ou insaturé, éventuellement aromatique, éventuellement substitué, comprenant éventuellement au moins un autre hétéroatome différent ou non de l'azote.
   - Un radical alkylcarbonylamino (R'CO-NR-) dans lequel le radical R est un atome d'hydrogène, un radical alkyle en C₁-C₄ et le radical R' représente un radical alkyle en C₁-C₄, un radical phényle ;
   - Un radical alkylsulfonyle (R-SO₂-) dans lequel le radical R représente un radical alkyle en C₁-C₄.
   - Un radical alkylsulfinyle (R-SO-) dans lequel le radical R représente un radical alkyle en C₁-C₄.
   - Un radical alkylcarbonyle (R-CO-) dans lequel le radical R représente un radical alkyle en C₁-C₄ ;
   Ces radicaux R étant éventuellement interrompus par un ou plusieurs hétéroatomes choisis parmi l'oxygène, l'azote et le soufre, substitué par au moins un groupement correspondant aux formules (a) et (b) suivantes : 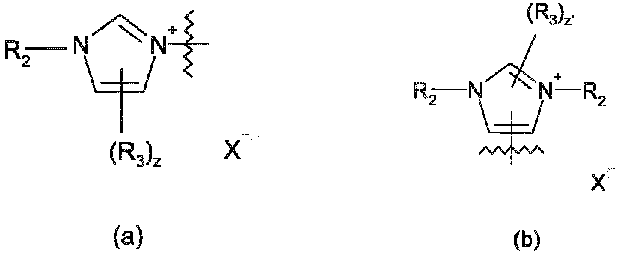 dans lesquelles le radical R₂ représente un radical alkyle en C₁-C₆ éventuellement substitué, le radical R₃ représente un atome d'halogène, un radical alkyle en C₁-C₆ éventuellement substitué, un radical monohydroxylalkyle en C₁-C₄, un radical alcoxyle en C₁-C₄, un radical hydroxycarbonyle, un radical alkyl(C₁-C₆)thiol, un radical amino disubstitué par un radical alkyle en C₁-C₄, z est un nombre entier compris entre 0 et 3 compris, z' est un nombre entier compris entre 0 et 2 compris,
- L est un bras de liaison cationique alkylène en C₂-C₂₀ interrompu par au moins un groupement correspondant aux formules suivantes : 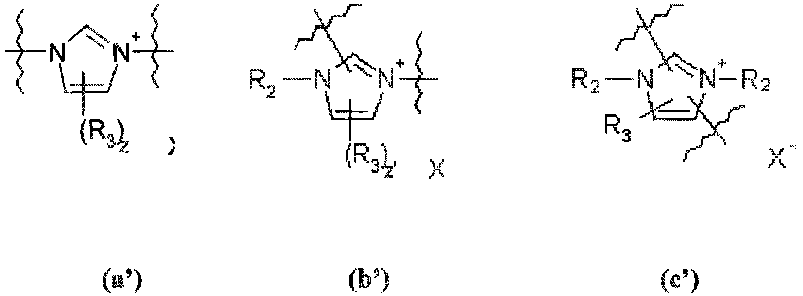 dans lesquelles le radical R₂ représente un radical alkyle en C₁-C₆ éventuellement substitué, le radical R₃ représente un atome d'halogène, un radical alkyle en C₁-C₆ éventuellement substitué, un radical monohydroxylalkyle en C₁-C₄, un radical alkoxy en C₁-C₄, un radical hydroxycarbonyl, un radical alkyl(C₁-C₆)thio, un radical amino disubstitué par un radical alkyle (C₁-C₄), z est un nombre entier compris entre 0 et 3 compris, z' est un nombre entier compris entre 0 et 2 compris,
   - X⁻ représente un contre-ion permettant d'assurer l'électroneutralité des composés de formule (I) à (IV) ;
   ainsi que leurs sels d'addition avec un acide et leurs solvates.

Les colorants naphtalimides selon la présente demande sont cationiques.

Les colorants de la présente invention permettent d'obtenir des colorations très chromatiques et qui présentent une bonne résistance vis-à-vis des agents extérieurs, notamment, des shampooings. Par ailleurs, les colorants de la présente invention permettent d'obtenir un effet éclaircissant sur des fibres kératiniques foncées, notamment des fibres kératiniques présentant une hauteur de ton inférieure ou égale à 6, de préférence, inférieure ou égale à 4, sans avoir recours à un agent oxydant, les fibres pouvant être colorées artificiellement ou naturellement. Lorsque l'utilisation d'un agent oxydant est souhaitée, par exemple pour obtenir une décoloration plus importante, les colorants de la présente invention présentent une bonne stabilité en milieu oxydant.

La présente demande porte également sur l'utilisation en tant que colorant direct, d'au moins un des colorants de type naphtalimide objets de la présente demande, pour la teinture des fibres kératiniques, et en particulier, des fibres kératiniques humaines telles que des cheveux.

La présente invention a de même pour objet des compositions tinctoriales comprenant, dans un milieu approprié pour la teinture, au moins l'un des colorants de formule (I) à (IV), ou leurs sels d'addition avec un acide, ou leurs solvates.

Elle concerne, de plus, un procédé de coloration de fibres kératiniques qui comprend l'application de la composition de l'invention sur des fibres kératiniques, pendant une durée suffisante pour obtenir la coloration souhaitée.

Elle a enfin pour objet un dispositif à plusieurs compartiments comprenant, dans un premier compartiment, la composition selon l'invention, et dans un second compartiment, une composition oxydante.

Au sens de la présente invention, et à moins qu'une indication différente ne soit donnée :
- Un radical alkyl(èn)e ou la partie alkyl(èn)e d'un radical est linéaire ou ramifiée.
- Un radical alkyl(èn)e ou la partie alkyl(èn)e d'un radical est dite "substituée" lorsqu'elle comprend au moins un substituant choisi parmi les groupements :
   - hydroxyle ;
   - alcoxyle en C₁-C₄, (poly)hydroxyalcoxyle en C₂-C₄ ;
   - amino, amino substitué par un ou deux groupements alkyle, identiques ou différents, en C₁-C₄, éventuellement porteurs d'au moins un groupement hydroxyle, alcoxyle en C₁-C₂, lesdits radicaux alkyle pouvant former avec l'atome d'azote auquel ils sont rattachés, un hétérocycle comprenant de 5 à 7 chaînons, saturé ou insaturé, éventuellement aromatique, éventuellement substitué, comprenant éventuellement au moins un autre hétéroatome différent ou non de l'azote ;
   - un radical alkylcarbonylamino (R'CO-NR-) dans lequel le radical R est un atome d'hydrogène, un radical alkyle en C₁-C₄ et le radical R' représente un radical alkyle en C₁-C₄, un radical phényle ;
   - un radical alkylsulfonyle (R-SO₂-) dans lequel le radical R représente un radical alkyle en C₁-C₄ ;
   - un radical alkylsulfinyle (R-SO-) dans lequel le radical R représente un radical alkyle en C₁-C₄ ;
   - un radical alkylcarbonyle (R-CO-) dans lequel le radical R représente un radical alkyle en C₁-C₄.
- Un radical (hétéro)cyclique, saturé ou non, aromatique ou non, ou la partie (hétéro)cyclique, saturée ou non, aromatique ou non, d'un radical est dite 'substituée' lorsqu'elle comprend au moins un substituant, de préférence porté par un atome de carbone, choisi parmi :
   - un radical alkyle en C₁-C₁₆, de préférence en C₁-C₈, éventuellement substitué ;
   - un atome d'halogène tel que le chlore, le fluor ou le brome ;
   - un groupement hydroxyle ;
   - un radical alcoxyle en C₁-C₄ ; un radical (poly)hydroxyalcoxyle en C₂-C₄ ;
   - un radical amino ;
   - un radical amino substitué par un ou deux radicaux alkyle identiques ou différents en C₁-C₄, éventuellement porteurs d'au moins un groupement hydroxyle, amino, (mono- ou di-)alkylamino en C₁-C₄ ou alcoxyle en C₁-C₂ ; les deux radicaux alkyle pouvant former avec l'atome d'azote auquel ils sont rattachés, un hétérocycle renfermant de 1 à 3 hétéroatomes, de préférence de 1 à 2 hétéroatomes, choisis parmi N, O, S, de préférence N, l'hétérocycle comprenant de 5 à 7 chaînons, étant saturé ou insaturé, aromatique ou non, et éventuellement substitué ;
   - un radical alkylcarbonylamino (R'CO-NR-) dans lequel le radical R est un atome d'hydrogène, un radical alkyle en C₁-C₄ et le radical R' est un radical alkyle en C₁-C₂ ;
   - un radical aminocarbonyle ((R)₂N-CO-) dans lequel les radicaux R, identiques ou non, représentent un atome d'hydrogène, un radical alkyle en C₁-C₄ ;
   - un radical alkylsulfonylamino ou arylsulfonylamino (R'S02-NR-) dans lequel le radical R représente un atome d'hydrogène ou un radical alkyle en C₁-C₄ et le radical R' représente un radical alkyle en C₁-C₄ ou un radical phényle ;
   - un radical aminosulfonyle ((R)₂N-SO₂-) dans lequel les radicaux R, identiques ou non, représentent un atome d'hydrogène ou un radical alkyle en C₁-C₄.

Les composés issus des formules (II) et (III) sont symétriques ou dissymétriques. Ils sont dits symétriques lorsqu'il existe un plan de symétrie perpendiculaire au bras de liaison L. Ils sont dits dissymétriques lorsqu'il n'existe pas de plan de symétrie perpendiculaire au bras de liaison L.

Dans ce qui va suivre et à moins qu'une autre indication ne soit donnée, les bornes délimitant un domaine de valeurs sont comprises dans ce domaine.

Comme indiqué auparavant, un premier objet de l'invention consiste en des composés correspondant aux formules (I) à (IV) précitées, leurs sels d'addition et leurs solvates.

De préférence, les composés de formule (I) à (IV) selon la présente invention sont tels que :
- les radicaux R₁, identiques ou non, indépendamment les uns des autres, représentent un atome d'hydrogène, un radical alkyle linéaire en C₁-C₄ éventuellement substitué par un groupement hydroxyle, un radical alcoxyle en C₁-C₂, un radical dialkyl(C₁-C₄)amino, un radical alkyl(C₁-C₃)carbonylamino ou un radical alkyl(C₁-C₂)sulfonylamino.

De manière encore plus préférée, les radicaux R₁, identiques ou différents, indépendamment les uns des autres, représentent :
- un atome d'hydrogène ;
- un radical alkyle en C₁-C₄, par exemple méthyle, éthyle.

L représente un bras de liaison cationique alkylène en C₂-C₂₀ interrompu par au moins un groupement correspondant aux formules suivantes : 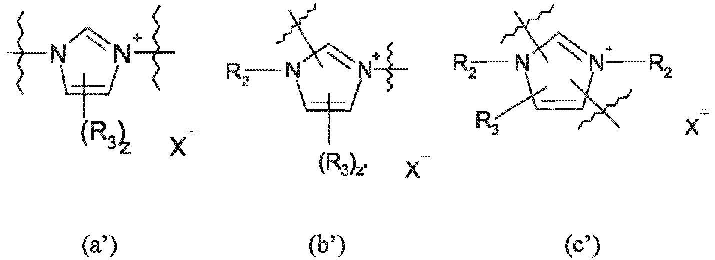 dans lesquelles :
- R₂ et R₃, pris séparément et indépendamment les uns des autres, ont la même signification que précédemment.
- z et z' ont la même signification que précédemment.
- X⁻ est tel que défini précédemment.

Le radical alkylène cationique ainsi défini peut être éventuellement interrompu par un ou plusieurs hétéroatomes ou groupements comprenant au moins un hétéroatome ou leurs combinaisons, comme par exemple l'oxygène, l'azote, un groupement -CO-, un groupement -SO-, un groupement -SO₂- ; à la condition qu'il n'y ait pas de groupe ou liaison azo, nitro, nitroso ou peroxo dans le bras de liaison L. Il peut aussi être éventuellement substitué, notamment par un ou plusieurs radicaux choisis parmi les radicaux hydroxyle, alcoxyle en C₁-C₂, (poly)hydroxyalcoxyle en C₂-C₄, amino substitué par un ou plusieurs groupements alkyle en C₁-C₂ éventuellement porteurs d'au moins un groupement hydroxyle.

Selon un mode de réalisation particulier des formules (a), (b), (b') et (c'), les radicaux R₂, pris séparément et indépendamment les uns des autres, représentent un radical alkyle en C₁-C₆, éventuellement substitué par un groupement hydroxyle, alcoxyle en C₁-C₂, hydroxycarbonyle, alkyl(C₁-C₆)thiol ou amino disubstitué par un radical alkyle en C₁-C₄.

Selon un mode de réalisation particulier des formules (a), (b), (b') et (c'), R₂ représente un radical alkyle tel que méthyle ou éthyle, un radical hydroxyalkyle tel que 2-hydroxyéthyle, un radical alcoxyalkyle tel que 2-méthoxyéthyle, un radical hydroxycarbonylalkyle tel que 3-hydroxycarbonylpropyle ou un radical dialkylaminoalkyle tel que 3-N,N-diméthylaminopropyle.

Selon un mode de réalisation particulier des formules (a), (b), (a'), (b') et (c'), R₃ représente un atome d'halogène choisi parmi le chlore et le fluor, un radical alkyle en C₁-C₆, un radical monohydroxylalkyle en C₁-C₄, un radical alcoxyle en C₁-C₄, un radical hydroxycarbonyle, un radical alkyl(C₁-C₆)thiol, un radical amino disubstitué par un radical alkyle en C₁-C₄. A titre d'exemple de radicaux R₃, on peut citer un atome de chlore, un méthyle, un éthyle, un 2-hydroxyéthyle, un méthoxy, un hydroxycarbonyle ou un diméthylamino.

Selon une variante des formules (a), (b), (a') et (b'), z et z' sont égaux à 0.

Selon un mode de réalisation particulier, les radicaux R, pris séparément et indépendamment les uns des autres, représentent un atome d'hydrogène ; un radical alkyle en C₁-C₆, de préférence en C₂-C₆, éventuellement substitué par un ou plusieurs groupements identiques ou différents de préférence choisis parmi les substituants hydroxyle, alcoxyle en C₁-C₂, et éventuellement substitué par au moins un groupement correspondant à la formule (a) telle que définie précédemment ; un radical aryle ou arylalkyle, tel que phényle, benzyle, la partie aryle étant éventuellement substituée par un ou plusieurs groupements identiques ou différents, de préférence choisis parmi par un atome de chlore, un groupement amino, un groupement hydroxyle, un groupement alcoxyle en C₁-C₂, un groupement amino mono ou disubstitué par deux radicaux alkyle en C₁-C₄ identiques ou différents éventuellement porteurs d'au moins un groupement hydroxyle.

Conformément à un mode de réalisation préféré de l'invention, les radicaux R, identiques ou différents, indépendamment l'un de l'autre, représentent un atome d'hydrogène, un radical alkyle en C₁-C₃ tel que méthyle ou éthyle, un radical hydroxyalkyle tel que 2-hydroxyéthyle, un radical alcoxyalkyle tel que 2-méthoxyéthyle, un radical alkyle en C₁-C₃, substitué par au moins un groupement correspondant à la formule (a") suivante, un radical benzyle éventuellement substitué par un ou plusieurs radicaux, identiques ou différents, choisi parmi les groupements hydroxyle, alcoxyle tel que méthoxy, amino, (di)alkylamino tel que diméthylamino, (di)(hydroxyalkyl)amino tel que (di)(2-hydroxyéthyl)amino.

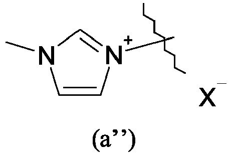

Dans les formules (I) à (IV), X⁻ représente un contre-ion organique ou minéral ou un mélange de contre-ions organiques ou minéraux, permettant d'équilibrer la ou les charges des composés de formule (I) à (IV). X⁻ est, par exemple, choisi parmi un halogénure tel que chlorure, bromure, fluorure, iodure ; un hydroxyde ; un sulfate ; un hydrogénosulfate ; un alkylsulfate pour lequel la partie alkyle, linéaire ou ramifiée, est en C₁-C₆, comme l'ion méthylsulfate ou éthylsulfate ; les carbonates et hydrogénocarbonates ; des sels d'acides carboxyliques tels que le formiate, l'acétate, le citrate, le tartrate, l'oxalate ; les alkylsulfonates pour lesquels la partie alkyle, linéaire ou ramifiée, est en C₁-C₆ comme l'ion méthylsulfonate ; les arylsulfonates pour lesquels la partie aryle, de préférence phényle, est éventuellement substituée par un ou plusieurs radicaux alkyle en C₁-C₄, tel que, par exemple, le 4-toluylsulfonate ; les alkylsulfonyles tel que le mésylate.

Les sels d'addition avec un acide des composés de formule (I) à (IV) peuvent être, à titre d'exemple, des sels d'addition avec un acide organique ou minéral tel que l'acide chlorhydrique, l'acide bromhydrique, l'acide sulfurique ou les acides (alkyl- ou phényl-) sulfoniques tels que l'acide p-toluènesulfonique ou l'acide méthylsulfonique.

Les solvates des composés de formule (I) à (IV) représentent les hydrates de tels composés et l'association d'un composé de formule (I) à (IV) avec un alcool, linéaire ou ramifié, en C₁-C₄ tel que le méthanol, l'éthanol, l'isopropanol, le n-propanol.

Conformément à un mode de réalisation particulier de l'invention, les composés correspondant aux formules (I), (II), (III) et (IV) ainsi qu'à leurs formes résonnantes et/ou leurs sels d'addition avec un acide et/ou leurs solvates sont choisis parmi les composés suivants :
- Le sel de 3-méthyl-1-{3-[(6-{[3-(3-méthyl-1H-imidazol-3-ium-1-yl)propyl]amino}-1,3-dioxo-1H-benzo[de]isoquinolin-2(3H)-yl]propyl}-1H-imidazol-3-ium: 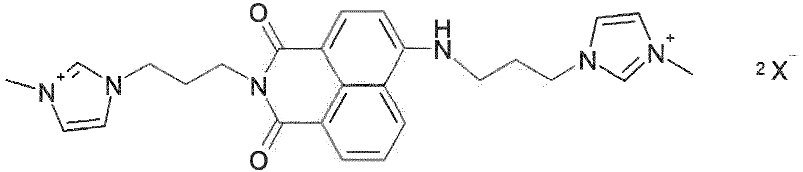
- Le sel de 3-{5-[(2-éthyl-1,3-dioxo-2,3-dihydro-1H-benzo[de]isoquinolin-6-yl)amino]pentyl}-1-[3-({2-[3-(1-méthyl-1H-imidazol-3-ium-3-yl)propyl]-1,3-dioxo-2,3-dihydro-1H-benzo[de]isoquinolin-6-yl}amino)propyl]-1H-imidazol-3-ium 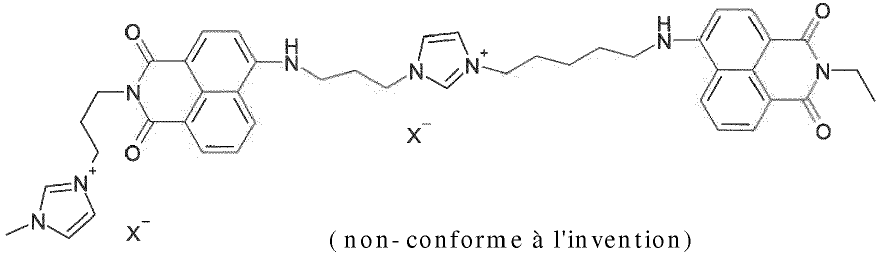
- Le sel de 1-méthyl-3-{3-[6-[(3-{3-[5-({2-[3-(1-méthyl-1H-imidazol-3-ium-3-yl)propyl]-1,3-dioxo-2,3-dihydro-1H-benzo[de]isoquinolin-6-yl}amino)pentyl]-1H-imidazol-3-ium-1-yl}propyl)amino]-1,3-dioxo-1H-benzo[de]isoquinolin-2(3H)-yl]-propyl}-1H-imidazol-3 -ium 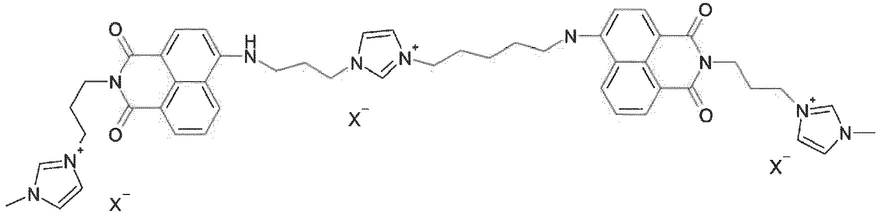
- Le sel de 3,3'-butane-1,4-diylbis{1-[5-({2-[3-(1-méthyl-1H-imidazol-3-ium-3-yl)propyl]-1,3-dioxo-2,3-dihydro-1H-benzo[de]isoquinolin-6-yl}amino)-pentyl]-1H-imidazol-3-ium} 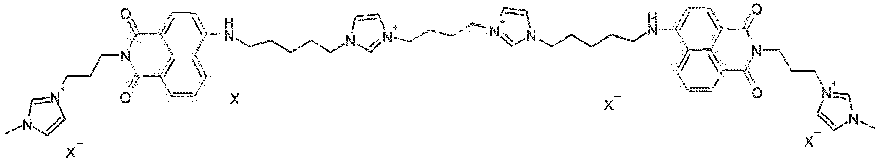
- Le sel de 1-méthyl-3-[3-({2-[3-(3-{5-[6-{[3-(1-méthyl-1H-imidazol-3-ium-3-yl)propyl]amino}-1,3-dioxo-1H-benzo[de]isoquinolin-2(3H)-yl]pentyl}-1H-imidazol-3-ium-1-yl)propyl]-1,3-dioxo-2,3-dihydro-1H-benzo[de]isoquinolin-6-yl}amino)propyl]-1H-imidazol-3-ium 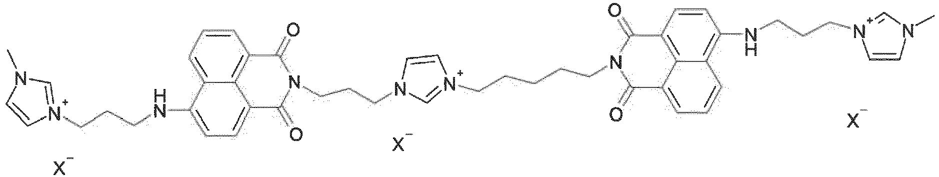
- Le sel de 1-méthyl-3-{3-[6-{[3-(3-{6-[6-{[3-(3-méthyl-1H-imidazol-3-ium-1-yl)propyl]amino}-1,3-dioxo-1H-benzo[de]isoquinolin-2(3H)-yl]hexyl}-1H-imidazol-3-ium-1-yl)propyl]amino}-1,3-dioxo-1H-benzo[de]isoquinolin-2(3H)-yl]propyl}-1H-imidazol-3 -ium 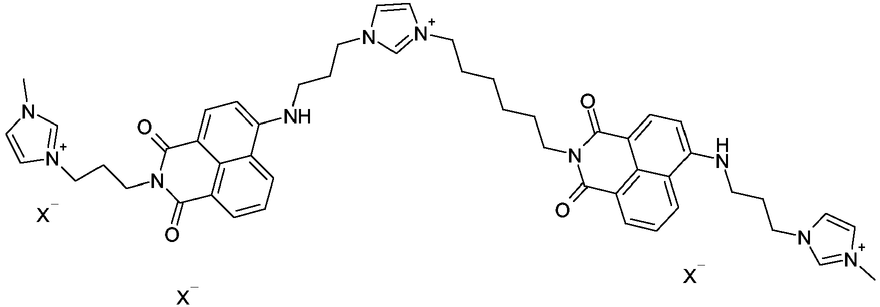
- Le sel de 3-méthyl-1-[3-({2-[6-(1-{6-[6-{[3-(1-méthyl-1H-imidazol-3-ium-3-yl)propyl]amino}-1,3-dioxo-1H-benzo[de]isoquinolin-2(3H)-yl]hexyl}-1H-imidazol-3-ium-3-yl)hexyl]-1,3-dioxo-2,3-dihydro-1H-benzo[de]isoquinolin-6-yl}-amino)propyl]-1H-imidazol-3-ium

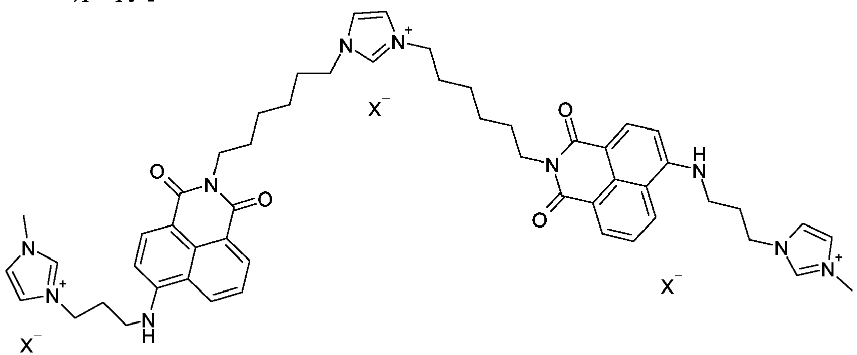

Selon un mode particulier de l'invention, les composés de formule (I) correspondent à la formule suivante : 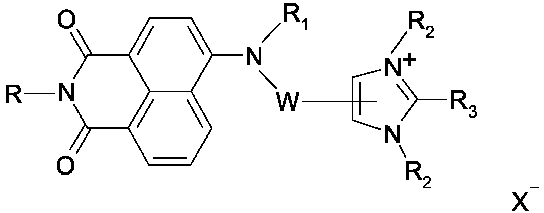 dans laquelle
- W représente un radical alkyle en C₁-C₈, de préférence, en C₂-C₈, et de manière encore plus préférée, en C₂-C₄,
- R, R₁, R₂, R₃, et X⁻ sont tels que définis précédemment.

De préférence, ils correspondent à la formule suivante : 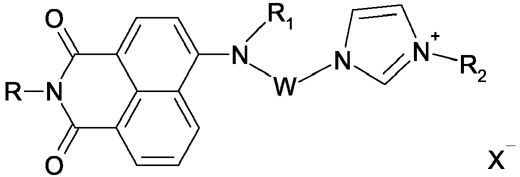 dans laquelle
- W représente un radical alkyle en C₁-C₈, de préférence, en C₂-C₈, et de manière encore plus préférée, en C₂-C₄,
- R, R₁, R₂ et X⁻ sont tels que définis précédemment.

Selon un mode de réalisation particulier de l'invention, les composés de formule (II) correspondent à la formule suivante : 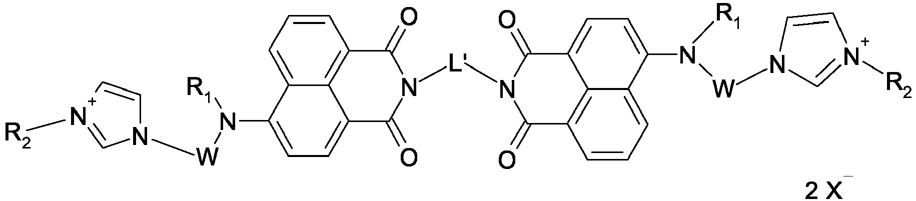 dans laquelle
- W représente un radical alkyle en C₁-C₈, de préférence, en C₂-C₈, et de manière encore plus préférée, en C₂-C₄,
- R₁, R₂ et X⁻ sont tels que définis précédemment,
- L' est défini comme L.

Selon un mode de réalisation particulier de l'invention, les composés de formule (III) correspondent à la formule suivante : 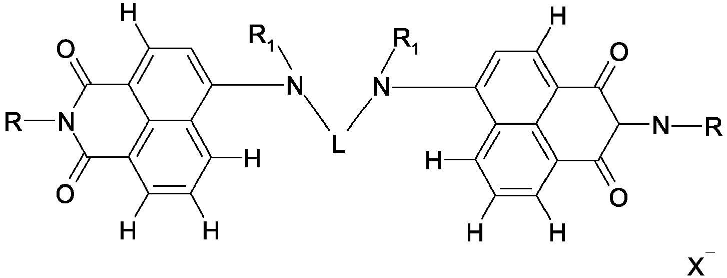 dans laquelle R, R₁ et L sont tels que définis précédemment.

Selon un mode de réalisation particulier de l'invention, les composés de formule (IV) correspondent à la formule suivante : 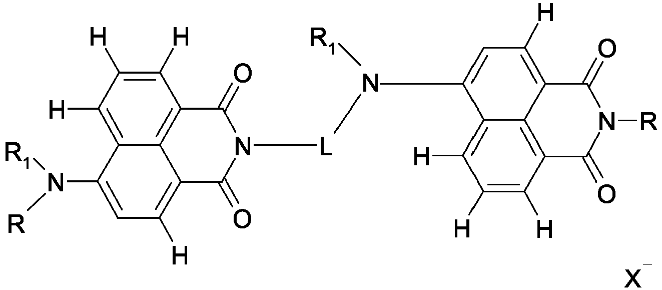 dans laquelle R, R₁, L et X⁻ sont tels que définis précédemment.

Les composés représentés précédemment peuvent notamment être obtenus à partir des procédés de préparation décrits, par exemple, dans les documents :
- Konstantinova, T. ; Spirieva, A. ; Petkova, T. Dyes and Pigments 2000, 45, 125-129,
- Gunnlaugsson, T. ; Kruger, P.E. ; Clive Lee, T. ; Parkesh, R.; Pfeffer, F.M.; Hussey, G.M. Tetrahedron Lett., 2003, 44, 6575-6578,
- Shepard, E. R.; Shonle, H.A. J. Am. Chem. Soc. 1947, 69, 2269-2270.

La composition tinctoriale selon l'invention peut contenir un ou plusieurs composés de type naphtalimide de formule (I), (II), (III) ou (IV), ou leurs sels d'addition avec un acide et/ou solvates, tels que définis précédemment, les mélanges de ces composés étant alors possibles en toutes proportions relatives, dans un milieu approprié pour la teinture.

La composition selon la présente demande comprend généralement de 0,001 à 10 %, de préférence 0,01 à 10 % en poids d'un ou plusieurs composés de type naphtalimide de formule (I), (II), (III) ou (IV), ou de leurs sels d'addition avec un acide et/ou solvates, par rapport au poids total de la composition.

La composition tinctoriale conforme à l'invention peut, en outre, contenir un ou plusieurs colorants directs additionnels autres que les colorants directs de formule (I), (II), (III) ou (IV), ces colorants directs additionnels pouvant notamment être choisis parmi les colorants nitrés de la série benzénique, neutres, acides ou cationiques, les colorants directs azoïques, neutres acides ou cationiques, les colorants directs quinonique, et en particulier, anthraquinonique, neutres, acides ou cationiques, les colorants directs azinique, les colorants directs triarylméthanique, les colorants directs indoaminique, les colorants de type tétraazapentaméthinique et les colorants directs naturels.

Parmi les colorants directs benzéniques utilisables selon l'invention, on peut citer de manière non limitative les composés suivants :
- 1,4-diamino-2-nitrobenzène,
- 1-amino-2 nitro-4-β-hydroxyéthylaminobenzène,
- 1-amino-2 nitro-4-bis(β-hydroxyéthyl)-aminobenzène,
- 1,4-bis(β-hydroxyéthylamino)-2-nitrobenzène,
- 1-β-hydroxyéthylamino-2-nitro-4-bis-(β-hydroxyéthylamino)-benzène,
- 1-β-hydroxyéthylamino-2-nitro-4-aminobenzène,
- 1-β-hydroxyéthylamino-2-nitro-4-(éthyl)(β-hydroxyéthyl)-aminobenzène,
- 1-amino-3-méthyl-4-β-hydroxyéthylamino-6-nitrobenzène,
- 1-amino-2-nitro-4-β-hydroxyéthylamino-5-chlorobenzène,
- 1,2-diamino-4-nitrobenzène,
- 1-amino-2-β-hydroxyéthylamino-5-nitrobenzène,
- 1,2-bis-(β-hydroxyéthylamino)-4-nitrobenzène,
- 1-amino-2-tris-(hydroxyméthyl)-méthylamino-5-nitrobenzène,
- 1-hydroxy-2-amino-5-nitrobenzène,
- 1-hydroxy-2-amino-4-nitrobenzène,
- 1-hydroxy-3 -nitro-4-aminobenzène,
- 1-hydroxy-2-amino-4,6-dinitrobenzène,
- 1-β-hydroxyéthyloxy-2-β-hydroxyéthylamino-5-nitrobenzène,
- 1-méthoxy-2-β-hydroxyéthylamino-5-nitrobenzène,
- 1-β-hydroxyéthyloxy-3-méthylamino-4-nitrobenzène,
- 1-β,γ-dihydroxypropyloxy-3-méthylamino-4-nitrobenzène,
- 1-β-hydroxyéthylamino-4-β,γ-dihydroxypropyloxy-2-nitrobenzène,
- 1-β,γ-dihydroxypropylamino-4-trifluorométhyl-2-nitrobenzène,
- 1-β-hydroxyéthylamino-4-trifluorométhyl-2-nitrobenzène,
- 1-β-hydroxyéthylamino-3-méthyl-2-nitrobenzène,
- 1-β-aminoéthylamino-5-méthoxy-2-nitrobenzène,
- 1-hydroxy-2-chloro-6-éthylamino-4-nitrobenzène,
- 1-hydroxy-2-chloro-6-amino-4-nitrobenzène,
- 1-hydroxy-6-bis-(β-hydroxyéthyl)-amino-3-nitrobenzène,
- 1-β-hydroxyéthylamino-2-nitrobenzène,
- 1-hydroxy-4-β-hydroxyéthylamino-3-nitrobenzène.

Parmi les colorants directs azoïques utilisables selon l'invention, on peut citer les colorants azoïques cationiques décrits dans les demandes de brevet WO 95/15144, WO 95/01772 et EP-A-714 954.

Parmi ces composés, on peut tout particulièrement citer les colorants suivants :
- le chlorure de 1,3-diméthyl-2-[[4-(diméthylamino)phényl]azo]-1H-imidazolium,
- le chlorure de 1,3-diméthyl-2-[(4-aminophényl)azo]-1H-imidazolium,
- le méthylsulfate de 1-méthyl-4-[(méthylphénylhydrazono)-méthyl]-pyridinium.

On peut également citer, parmi les colorants directs azoïque, les colorants suivants, décrits dans le COLOUR INDEX INTERNATIONAL, 3^{e} édition :
- Disperse Red 17,
- Acid Yellow 9,
- Acid Black 1,
- Basic Red 22,
- Basic Red 76,
- Basic Yellow 57,
- Basic Brown 16,
- Acid Yellow 36,
- Acid Orange 7,
- Acid Red 33,
- Acid Red 35,
- Basic Brown 17,
- Acid Yellow 23,
- Acid Orange 24,
- Disperse Black 9.

On peut aussi citer le 1-(4'-aminodiphénylazo)-2-méthyl-4bis-(β-hydroxy-éthyl)aminobenzène et l'acide 4-hydroxy-3-(2-méthoxyphénylazo)-1-naphtalène sulfonique.

Parmi les colorants directs quinonique, on peut citer les colorants suivants :
- Disperse Red 15,
- Solvent Violet 13,
- Acid Violet 43,
- Disperse Violet 1,
- Disperse Violet 4,
- Disperse Blue 1,
- Disperse Violet 8,
- Disperse Blue 3,
- Disperse Red 11,
- Acid Blue 62,
- Disperse Blue 7,
- Basic Blue 22,
- Disperse Violet 15,
- Basic Blue 99,
ainsi que les composés suivants :
- 1-N-méthylmorpholiniumpropylamino-4-hydroxyanthraquinone,
- 1-aminopropylamino-4-méthylaminoanthraquinone,
- 1-aminopropylaminoanthraquinone,
- 5-β-hydroxyéthyl-1,4-diaminoanthraquinone,
- 2-aminoéthylaminoanthraquinone,
- 1,4-bis-(P,γ-dihydroxypropylamino)-anthraquinone.

Parmi les colorants aziniques, on peut citer les composés suivants :
- Basic Blue 17,
- Basic Red 2.

Parmi les colorants triarylméthaniques utilisables selon l'invention, on peut citer les composés suivants :
- Basic Green 1,
- Acid blue 9,
- Basic Violet 3,
- Basic Violet 14,
- Basic Blue 7,
- Acid Violet 49,
- Basic Blue 26,
- Acid Blue 7.

Parmi les colorants indoaminiques utilisables selon l'invention, on peut citer les composés suivants :
- la 2-β-hydroxyéthylamino-5-[bis-(β-4'-hydroxyéthyl)amino]anilino-1,4-benzoquinone,
- la 2-β-hydroxyéthylamino-5-(2'-méthoxy-4'-amino)anilino-1,4-benzoquinone,
- la 3-N(2'-chloro-4'-hydroxy)phénylacétylamino-6-méthoxy-1,4-benzoquinone imine,
- la 3-N(3'-chloro-4'-méthylamino)phényluréido-6-méthyl-1,4-benzoquinone imine,
- la 3-[4'-N-(éthyl,carbamylméthyl)amino]-phényl-uréido-6 méthyl-1,4-benzoquinone imine.

Parmi les colorants de type tétraazapentaméthinique utilisables selon l'invention, on peut citer les composés suivants figurant dans le tableau ci-dessous, An représentant en général un anion organique ou minéral par exemple choisi parmi un halogénure tel que un chlorure, un bromure, un fluorure, un iodure ; un hydroxyde ; un sulfate ; un hydrogénosulfate ; un alkyl(C₁-C₆)sulfate tel que par exemple un méthylsulfate ou un éthylsulfate ; un acétate ; un tartrate ; un oxalate ; un alkyl(C₁-C₆)sulfonate tel que un méthylsulfonate ; un arylsulfonate substitué ou non substitué par un radical alkyle en C₁-C₄ tel que par exemple un 4-toluylsulfonate. An est de préférence un chlorure ; un méthylsulfate :

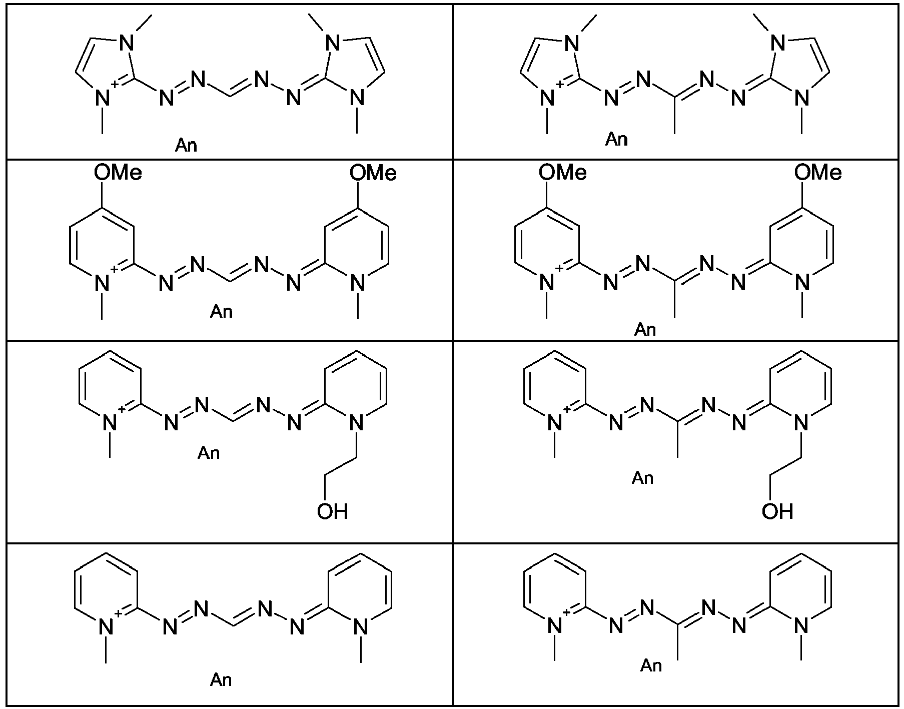

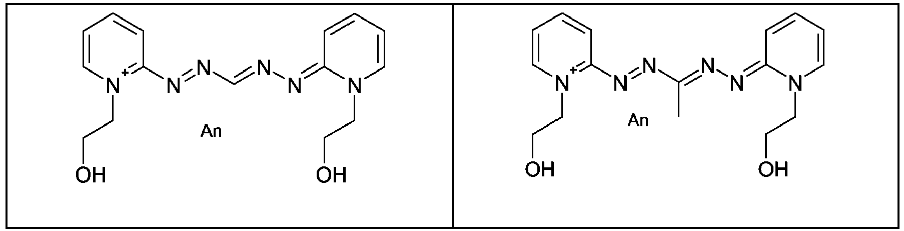

Parmi les colorants directs naturels utilisables selon l'invention, on peut citer la lawsone, la juglone, l'alizarine, la purpurine, l'acide carminique, l'acide kermésique, la purpurogalline, le protocatéchaldéhyde, l'indigo, l'isatine, la curcumine, la spinulosine, l'apigénidine. On peut également utiliser les extraits ou décoctions contenant ces colorants naturels et notamment les cataplasmes ou extraits à base de henné.

Le ou les colorants directs additionnels représentent de préférence de 0,001 à 20 % en poids environ du poids total de la composition prête à l'emploi et encore plus préférentiellement de 0,005 à 10 % en poids environ.

La composition de la présente invention peut, en outre, comprendre un ou plusieurs précurseurs de colorant d'oxydation : une ou plusieurs bases d'oxydation et/ou un ou plusieurs coupleurs.

A titre d'exemple, les bases d'oxydation sont choisies parmi les paraphénylènediamines, les bis-phénylalkylènediamines, les para-aminophénols, les ortho-aminophénols, les bases hétérocycliques autres que les paraphénylènediamines hétérocycliques et leurs sels d'addition.

Parmi les paraphénylènediamines, on peut citer à titre d'exemple, la paraphénylènediamine, la paratoluylènediamine, la 2-chloroparaphénylènediamine, la 2,3-diméthyl paraphénylènediamine, la 2,6-diméthylparaphénylènediamine, la 2,6-diéthyl paraphénylènediamine, la 2,5-diméthylparaphénylènediamine, la N,N-diméthyl paraphénylènediamine, la N,N-diéthylparaphénylènediamine, la N,N-dipropyl paraphénylènediamine, la 4-amino N,N-diéthyl-3-méthylaniline, la N,N-bis-(β-hydroxyéthyl)paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoroparaphénylènediamine, la 2-isopropyl paraphénylène-diamine, la N-(β-hydroxypropyl)paraphénylènediamine, la 2-hydroxyméthylpara-phénylènediamine, la N,N-diméthyl 3-méthyl paraphénylènediamine, la N,N-(éthyl, β-hydroxyéthyl)paraphénylènediamine, la N-(β,γ-dihydroxypropyl)paraphénylène-diamine, la N-(4'-aminophényl) paraphénylènediamine, la N-phényl paraphénylène-diamine, la 2-β-hydroxyéthyloxy paraphénylènediamine, la 2-β-acétylaminoéthyloxy paraphénylènediamine, la N-(β-méthoxyéthyl)paraphénylène-diamine, la 4-amino-phénylpyrrolidine, la 2-thiényl paraphénylènediamine, le 2-β hydroxyéthylamino 5-amino toluène, la 3-hydroxy 1-(4'-aminophényl)pyrrolidine et leurs sels d'addition avec un acide.

Parmi les paraphénylènediamines citées ci-dessus, la paraphénylènediamine, la paratoluylènediamine, la 2-isopropyl paraphénylènediamine, la 2-β-hydroxyéthyl paraphénylènediamine, la 2-β-hydroxyéthyloxy paraphénylène-diamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide sont particulièrement préférées.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diaminophénoxy)-3,6-dioxaoctane, et leurs sels d'addition.

Parmi les para-aminophénols, on peut citer à titre d'exemple, le para-aminophénol, le 4-amino-3-méthylphénol, le 4-amino 3-fluorophénol, le 4-amino-3-chlorophénol, le 4-amino-3-hydroxyméthylphénol, le 4-amino-2-méthylphénol, le 4-amino-2-hydroxyméthylphénol, le 4-amino-2-méthoxyméthylphénol, le 4-amino-2-aminométhylphénol, le 4-amino-2-(β-hydroxyéthylaminométhyl) phénol, le 4-amino-2-fluorophénol, et leurs sels d'addition avec un acide.

Parmi les ortho-aminophénols, on peut citer à titre d'exemple, le 2-amino-phénol, le 2-amino-5-méthylphénol, le 2-amino-6-méthylphénol, le 5-acétamido-2-aminophénol, et leurs sels d'addition.

Parmi les bases hétérocycliques, on peut citer à titre d'exemple, les dérivés pyridiniques, les dérivés pyrimidiniques et les dérivés pyrazoliques.

Parmi les dérivés pyridiniques, on peut citer les composés décrits par exemple dans les brevets GB-A-1 026 978 et GB-A- 1 153 196, comme la 2,5-diamino pyridine, la 2-(4-méthoxyphényl)amino 3-amino pyridine, la 3,4-diamino pyridine, et leurs sels d'addition.

D'autres bases d'oxydation pyridiniques utiles dans la présente invention sont les bases d'oxydation 3-amino pyrazolo-[1,5-a]-pyridines ou leurs sels d'addition décrits par exemple dans la demande de brevet FR-A-2 801 308. A titre d'exemple, on peut citer la pyrazolo[1,5-a]pyridin-3-ylamine ; la 2-acétylamino pyrazolo-[1,5-a] pyridin-3-ylamine ; la 2-morpholin-4-yl-pyrazolo[1,5-a]pyridin-3-ylamine ; l'acide 3-amino-pyrazolo[1,5-a]pyridin-2-carboxylique ; la 2-méthoxy-pyrazolo[1,5-a]pyridine-3-ylamino ; le (3-amino-pyrazolo[1,5-a]pyridine-7-yl)-méthanol ; le 2-(3-amino-pyrazolo[1,5-a]pyridine-5-yl)-éthanol ; le 2-(3-amino-pyrazolo[1,5-a]pyridine-7-yl)-éthanol ; le (3-amino-pyrazolo[1,5-a]pyridine-2-yl)-méthanol ; la 3,6-diamino-pyrazolo[1,5-a]pyridine ; la 3,4-diamino-pyrazolo[1,5-a]pyridine ; la pyrazolo[1,5-a]-pyridine-3,7-diamine ; la 7-morpholin-4-yl-pyrazolo[1,5-a]pyridin-3-ylamine ; la pyrazolo[1,5-a]pyridine-3,5-diamine ; la 5-morpholin-4-yl-pyrazolo[1,5-a]pyridin-3-ylamine ; le 2-[(3-amino-pyrazolo[1,5-a]pyridin-5-yl)-(2-hydroxyéthyl)-amino]-éthanol ; le 2-[(3-amino-pyrazolo[1,5-a]pyridin-7-yl)-(2-hydroxyéthyl)-amino]-éthanol ; la 3-amino-pyrazolo[1,5-a]pyridine-5-ol ; 3-amino-pyrazolo[1,5-a]pyridine-4-ol ; la 3-amino-pyrazolo[1,5-a]pyridine-6-ol ; la 3-amino-pyrazolo[1,5-a]pyridine-7-ol ; ainsi que leurs sels d'addition.

Parmi les dérivés pyrimidiniques, on peut citer les composés décrits par exemple dans les brevets DE-A-23 59 399 ; JP 88-169571 ; EP-A-0 770 375 ou demande de brevet WO 96/15765 comme la 2,4,5,6-tétra-aminopyrimidine, la 4-hydroxy-2,5,6-triaminopyrimidine, la 2-hydroxy-4,5,6-triaminopyrimidine, la 2,4-dihydroxy-5,6-diaminopyrimidine, la 2,5,6-triaminopyrimidine, leurs sels d'addition et leurs formes tautomères, lorsqu'il existe un équilibre tautomérique.

Parmi les dérivés pyrazoliques, on peut citer les composés décrits dans les brevets DE-A-38 43 892, DE-A-41 33 957 et demandes de brevet WO 94/08969, WO 94/08970, FR-A-2 733 749 et DE-A-195 43 988 comme le 4,5-diamino-1-méthyl pyrazole, le 4,5-diamino-1-(β-hydroxyéthyl) pyrazole, le 3,4-diaminopyrazole, le 4,5-diamino-1-(4'-chlorobenzyl)pyrazole, le 4,5-diamino-1,3-diméthylpyrazole, le 4,5-diamino-3-méthyl-1-phényl pyrazole, le 4,5-diamino-1-méthyl 3-phényl pyrazole, le 4-amino-1,3-diméthyl 5-hydrazinopyrazole, le 1-benzyl-4,5-diamino-3-méthyl pyrazole, le 4,5-diamino-3-tert-butyl 1-méthylpyrazole, le 4,5-diamino-1-tert-butyl-3-méthyl pyrazole, le 4,5-diamino-1-(β-hydroxyéthyl)-3-méthylpyrazole, le 4,5-diamino-1-éthyl 3-méthylpyrazole, le 4,5-diamino-1-éthyl-3-(4'-méthoxyphényl)pyrazole, le 4,5-diamino 1-éthyl-3-hydroxyméthylpyrazole, le 4,5-diamino-3-hydroxyméthyl-1-méthylpyrazole, le 4,5-diamino-3-hydroxyméthyl-1-isopropylpyrazole, le 4,5-diamino-3-méthyl 1-isopropylpyrazole, le 4-amino-5-(2'-aminoéthyl)amino-1,3-diméthylpyrazole, le 3,4,5-triaminopyrazole, le 1-méthyl-3,4,5-triaminopyrazole, le 3,5-diamino-1-méthyl-4-méthylaminopyrazole, le 3,5-diamino-4-(β-hydroxyéthyl)amino-1-méthylpyrazole, et leurs sels d'addition.

La ou les bases d'oxydation présentes dans la composition de l'invention sont, en général, présentes en des quantités allant de 0,001 à 20 % en poids environ du poids total de la composition tinctoriale, de préférence, allant de 0,005 à 6 % en poids.

La composition selon l'invention contient, de préférence, un ou plusieurs coupleurs conventionnellement utilisés pour la teinture des fibres kératiniques. Parmi ces coupleurs, on peut notamment citer les métaphénylènediamines, les métadiphénols, les coupleurs naphtaléniques, les coupleurs hétérocycliques, ainsi que leurs sels d'addition.

A titre d'exemple, on peut citer le 1,3-dihydroxy benzène, le 1,3-dihydroxy-2-méthylbenzène, le 4-chloro-1,3-dihydroxybenzène, le 2,4-diamino-1-(β-hydroxyéthyl-oxy)benzène, le 2-amino-4-(β-hydroxyéthylamino)-1-méthoxybenzène, le 1,3-diamino-benzène, le 1,3-bis-(2,4-diaminophénoxy)propane, la 3-uréido aniline, le 3-uréido-1-diméthylaminobenzène, le sésamol, le 1-β-hydroxyéthylamino-3,4-méthylènedioxy-benzène, l'α-naphtol, le 2 méthyl-1-naphtol, le 6-hydroxy indole, le 4-hydroxyindole, le 4-hydroxy-N-méthylindole, la 2-amino-3-hydroxypyridine, la 6- hydroxy-benzomorpholine la 3,5-diamino-2,6-diméthoxypyridine, le 1-N-(β-hydroxyéthyl)-amino-3,4-méthylène dioxybenzène, le 2,6-bis-(β-hydroxyéthylamino)toluène et leurs sels d'addition.

Dans la composition de la présente invention, le ou les coupleurs sont généralement présents en des quantités allant de 0,001 à 20 % en poids environ du poids total de la composition tinctoriale, de préférence, allant de 0,005 à 6 % en poids.

D'une manière générale, les sels d'addition des bases d'oxydation et des coupleurs utilisables dans le cadre de l'invention sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates, et les sels d'addition avec une base telles que la soude, la potasse, l'ammoniaque, les amines ou les alcanolamines.

Le milieu approprié pour la teinture appelé aussi support de teinture est notamment approprié pour la teinture des fibres kératiniques telles que les cheveux. Un tel milieu est un milieu cosmétique généralement constitué par de l'eau ou par un mélange d'eau et d'au moins un solvant organique pour solubiliser les composés qui ne seraient pas suffisamment solubles dans l'eau. A titre de solvant organique, on peut, par exemple, citer les alcanols inférieurs en C₁-C₄, tels que l'éthanol et l'isopropanol ; les polyols et éthers de polyols comme le 2-butoxyéthanol, le propylèneglycol, le glycérol, le monométhyléther de propylèneglycol, le monoéthyléther et le monométhyléther du diéthylèneglycol ; ainsi que les alcools aromatiques comme l'alcool benzylique ou le phénoxyéthanol ; et leurs mélanges.

Les solvants sont présents dans des proportions comprises, de préférence, entre 1 et 40 % en poids environ, et encore plus préférentiellement, entre 5 et 30 % en poids environ, par rapport au poids total de la composition tinctoriale.

La composition tinctoriale conforme à l'invention peut également renfermer divers adjuvants utilisés classiquement dans les compositions pour la teinture des cheveux, tels que des agents tensio-actifs anioniques, cationiques, non-ioniques, amphotères, zwitterioniques ou leurs mélanges, des polymères anioniques, cationiques, non-ioniques, amphotères, zwitterioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrant, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiant.

Les adjuvants ci-dessus sont en général présents en des quantités allant, pour chacun d'eux, de 0,01 et 20 % en poids par rapport au poids total de la composition.

Selon une variante, la composition de l'invention comprend un agent oxydant afin d'obtenir un éclaircissement des fibres. Les agents oxydants classiquement utilisés pour la teinture d'oxydation des fibres kératiniques sont par exemple le peroxyde d'hydrogène, le peroxyde d'urée, les bromates de métaux alcalins, les persels tels que les perborates et persulfates, les peracides et les enzymes oxydases parmi lesquelles on peut citer les peroxydases, les oxydo-réductases à 2 électrons telles que les uricases et les oxygénases à 4 électrons comme les laccases. Selon un mode de réalisation particulier, la composition contient un agent oxydant de type peroxyde et/ou un agent oxydant de type persels, par exemple un mélange de peroxyde d'hydrogène et de persulfate, le peroxyde d'hydrogène seul ou le persulfate seul.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule suivante : 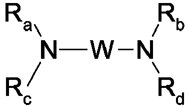 dans laquelle W est un reste propylène éventuellement substitué par un groupement hydroxyle ou un radical alkyle en C₁-C₄ ; Rₐ, R_{b}, R_{c}, et R_{d}, identiques ou différents, représentent un atome d'hydrogène, un radical alkyle en C₁-C₄ ou hydroxyalkyle en C₁-C₄.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

Le procédé de la présente invention est un procédé dans lequel on applique, sur les fibres, la composition selon la présente invention, telle que définie précédemment.

Selon un mode de réalisation particulier, le procédé de l'invention est mis en oeuvre en présence d'un agent oxydant. Cet agent oxydant est appliqué sur les fibres kératiniques pendant un temps suffisant pour obtenir l'éclaircissement souhaité. L'agent oxydant peut être présent dans la composition comprenant le composé de type naphtalimide, il peut être ajouté juste au moment de l'emploi ou il peut être appliqué simultanément ou séquentiellement à la composition contenant le composé de type naphtalimide.

Selon une variante, le procédé de l'invention comprend l'application de la composition de l'invention sur des fibres kératiniques foncées, notamment des cheveux présentant une hauteur de ton inférieure ou égale à 6, de préférence, inférieure ou égale à 4. Par « hauteur de ton », est entendu la classification classiquement usitée dans le domaine de la coloration capillaire défini dans l'ouvrage de C. Zviak, Science des traitement capillaires, Masson Ed., Paris, p. 278 (1988) ;
L'invention a enfin pour objet un kit de coloration des fibres kératiniques comprenant une composition comprenant d'une part, au moins l'un des colorants naphtalimide de formule (I), (II), (III) ou (IV), sels d'addition avec un acide et/ou solvates précités, et d'autre part, un agent oxydant.

Les exemples qui suivent servent à illustrer l'invention sans toutefois présenter un caractère limitatif.

### EXEMPLES

### Exemple 1 : Synthèse du composé de référence : le 3-méthyl-1-{3-[(2-méthyl-1,3 -dioxo-2,3 -dihydro-1H-benzo[de]isoquinolin-6-yl)amino]propyl}-1H-imidazol-3-ium méthyl-sulfate :

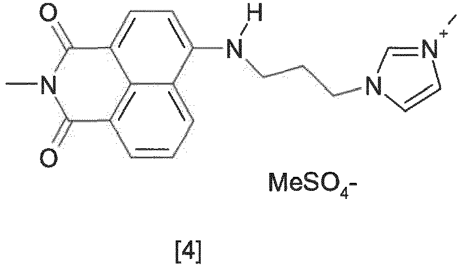

### Schéma de synthèse

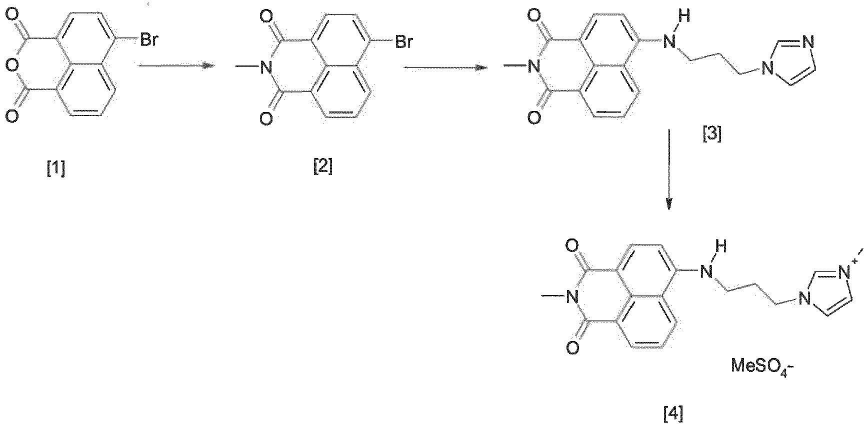

### Etape 1

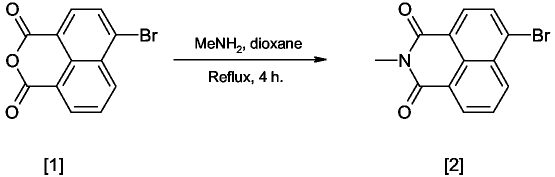

A une solution de 2,5 g d'anhydride 4-bromo-1,8-naphtalique [1] dans 45 mL de dioxane est ajouté 0,76 mL de méthylamine. Le mélange réactionnelle est chauffé à reflux pendant 4 h. Après refroidissement à température ambiante, le milieu réactionnel est versé dans 50 mL d'un mélange eau/glace. Une poudre jaune/orangé précipite immédiatement, elle est filtrée sur Büchner, puis dissoute dans un minimum de dichlorométhane avant d'être séchée sur sulfate de magnésium. Après filtration et évaporation, 2,4 g de composé attendu [2] sont récupérés sous forme d'une poudre jaune.

Les analyses sont conformes au produit attendu.

### Etape 2

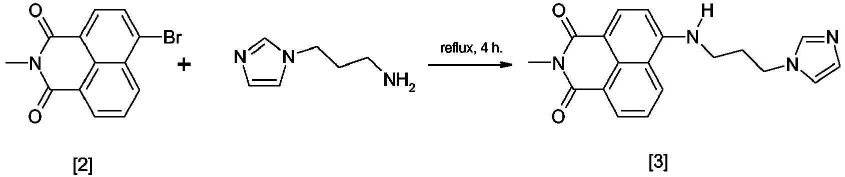

Une solution de 8 g de composé [2] dans 20 mL de 1-(3-amino-propyl)imidazole est plongée dans un bain préchauffé à 120 °C. Le milieu est chauffé pendant 1 h. Le milieu réactionnel est ramené à température ambiante, puis hydrolysé avec 70 mL d'eau distillée. L'eau se colore et un précipité orange commence à se former. Après avoir laissé reposer la solution pendant 12 h à 4 °C, le mélange est filtré sur Büchner. La poudre obtenue est travaillée avec plusieurs volumes de dichlorométhane et d'acétone, puis séchée sous vide. 6,2 g de composé attendu [3] sont récupérés sous forme d'une poudre jaune.

Les analyses sont conformes au produit attendu.

### Etape 3

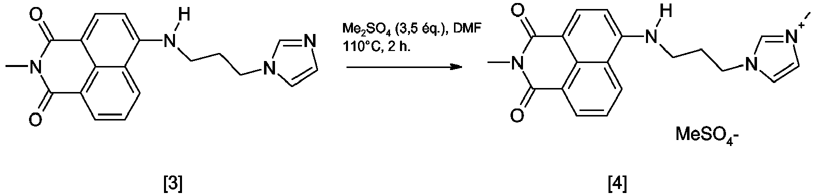

Une suspension de 7,3 g de composé [3] dans 24 ml de DMF, placée sous argon, est chauffée dans un bain d'huile préchauffée à 110 °C pendant 5 minutes. Après que le milieu réactionnel soit revenu à température ambiante, 3 ml de diméthylsulfate sont additionnés au goutte à goutte. Le milieu est chauffé pendant 2 h à 110 °C. Le mélange réactionnel est ensuite soumis à plusieurs co-évaporations avec du toluène afin d'éliminer la DMF. Le résidu brun obtenu est repris dans un minimum de méthanol auquel on ajoute de l'acétate d'éthyle jusqu'à obtention d'un précipité. Après filtration sous argon, la poudre jaune est travaillée dans l'acétate d'éthyle (1 h), puis l'éther (1 h) et enfin le chloroforme (30 minutes). Après filtration puis séchage, 9 g de composé attendu [4] sont récupérés sous forme d'une poudre jaune.

Les analyses sont conformes au produit attendu.
RMN 1H (MeOD) : 2,3ppm, m, 2H ; 3,8ppm, s, 3H; 3,6ppm, s, 3H ; 3,4ppm, s, 3H ; 3,5ppm, t, 2H, 4,4ppm ,t, 2H; 6,8ppm, d, 1H; 7,3ppm, s, 1H ; 7,5ppm, d, 1H; 7,8ppm, d, 1H ; 8,3ppm, d, 1H ; 8,5ppm ,d, 2H ; 9,0ppm, s, 1H

### Exemple 2 : Synthèse du 3-méthyl-1-{3-[(6-{[3-(3-méthyl-1H-imidazol-3-ium-1-yl)propyl]amino} 1,3-dioxo-1H-benzo[de]isoquinolin-2(3H)-yl]propyl}-1H-imidazol-3-ium bis(méthyl sulfate)

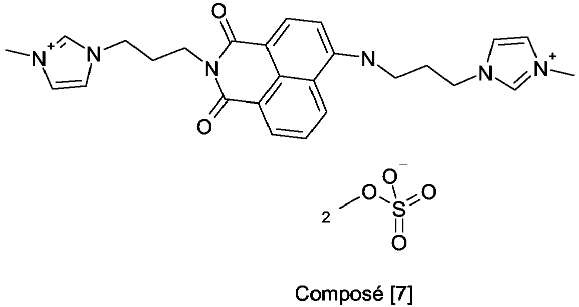

### Schéma de synthèse

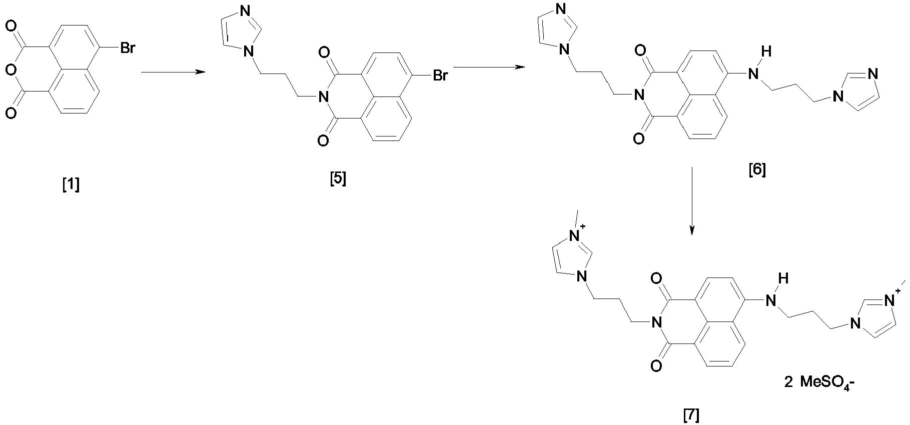

### Etape 1

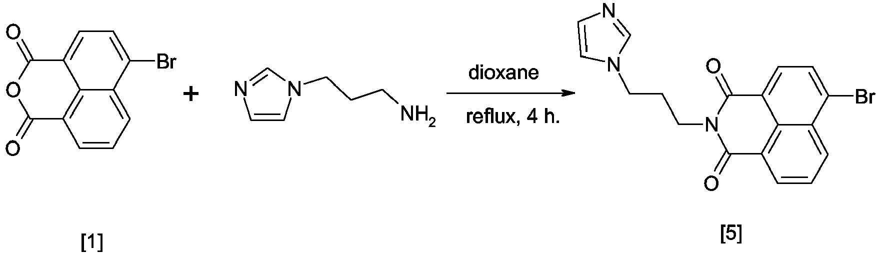

A une solution brune de 5,67 g d'anhydride 4-bromo-1,8-naphatalique [1] (20,5 mmol ; 1 éq) dans 90 ml de dioxane sont ajoutés 2,44 ml de 1-(3-aminopropyl)-imidazole [2] (20,5 mmol ; 1 éq). Le mélange réactionnel est chauffé à reflux du dioxane pendant 4 h.

Après refroidissement du milieu réactionnel à température ambiante, celui-ci est versé dans 100 ml d'un mélange eau/glace. Un précipité se forme. Celui-ci est filtré, puis dissout dans quelques millilitres de dichlorométhane avant d'être séché sur sulfate de magnésium. Après filtration et évaporation, une poudre jaunâtre est obtenue. Après purification par chromatographie sur colonne de silice (éluant dichlorométhane/acétone 1/1), 4,73 g d'une poudre jaune ocre correspondant au composé [5] sont obtenus.

Les analyses sont conformes au produit attendu.

### Etape 2

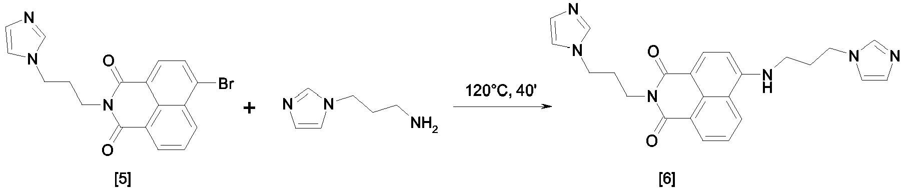

4,73 g de composé [5] (12,3 mmol ; 1 éq) sont dissouts par léger chauffage dans 8,8 ml de 1-(3-aminopropyl)imidazole (73,7 mmol ; 6 éq). Le mélange réactionnel est chauffé à 120 °C pendant 40 minutes. Le résidu noirâtre obtenu est ramené à température ambiante. 60 ml d'eau distillée sont ajoutés à ce résidu puis mis sous agitation pendant 5 minutes. La phase aqueuse est extraite avec du dichlorométhane.

La phase organique est par la suite lavée au chlorure d'ammonium, séchée sur sulfate de sodium filtrée, puis concentrée sous vide.

4,83 g d'une poudre orange correspondant au composé [6] sont obtenus.

Les analyses sont conformes au produit attendu.

### Etape 3

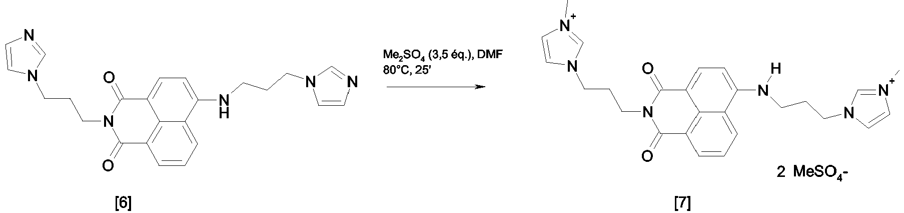

Une suspension de 4,83 g de composé [6] (11,2 mmol ; 1 éq) dans 10 ml de DMF est chauffée à 80 °C. 3,7 ml de diméthylsulfate (39,4 mmol ; 3,5 éq) sont ensuite additionnés au goutte à goutte au milieu réactionnel. Le mélange réactionnel est chauffé à 80 °C pendant 25 minutes puis ramené à température ambiante.

Celui-ci est ensuite soumis à plusieurs co-évaporations avec du toluène afin d'éliminer la DMF. Le résidu brun obtenu est repris six fois par un mélange constitué de méthanol (10 ml), de dichlorométhane (50 ml), et de cyclohexane (50 ml) et placé sous forte agitation pendant 5 minutes. Le surnageant est systématiquement éliminé.

Après concentration, puis séchage sous vide, 5,20 g d'une poudre jaune foncé correspondant au composé [7] sont obtenus.

Les analyses sont conformes au produit attendu.

### Exemples de teintures :

| | | |
|---|---|---|
| Colorant 1 (composé [7]) | 3-méthyl-1-{3-[(2-méthyl-1,3-dioxo-2,3-dihydro-1H-benzo[de]isoquinolin-6-yl)amino]propyl}-1H-imidazol-3-ium méthylsulfate | |
| Colorant 2 (composé [4]) | 3-méthyl-1-{3-[(6-{[3-(3-méthyl-1H-imidazol-3-ium-1-yl)propyl]amino}-1,3-dioxo-1H-benzo[de]isoquinolin-2(3H)-yl]propyl}-1H-imidazol-3-ium bis(méthylsulfate) | |
| Colorant 3 (comparatif) | 2-hydroxyéthylamino-5-nitroanisole | |
| Colorant 4 (comparatif) | Basic Yellow 87 : | 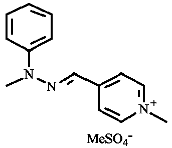 |

On a préparé quatre solutions tamponnées à pH 9 par mélange de 2 g d'acétate d'ammonium dans 40 ml d'eau. Le pH est ajusté par addition d'ammoniaque et le volume est complété à 100 ml par addition d'eau désionisée.

On dissout chacun des colorants 1 à 4 dans une solution tamponnée pour obtenir une concentration en colorant à 5.10⁻⁴ mol%.

On met en contact une mèche de cheveux blancs avec la solution colorante résultante selon un rapport de bain de 10 pour 1 (1 g de mèche pour 10g de solution).

Après 20 minutes de pause, la mèche est rincée à l'eau déionisée pour éliminer l'excès de solution de colorant.

Avec chacune des solutions colorantes, on obtient une mèche de cheveux jaunes. Les colorants 1 et 2 selon l'invention sont visiblement plus chromatiques. Lorsque la composition contenant les colorants 1 et 2 selon l'invention est appliquée sur cheveux foncés (hauteur de ton égale à 4), on obtient un effet optique éclaircissant.

### Test au shampooing

Chaque mèche de cheveux colorés selon l'étape précédente est lavée avec une solution comprenant 1 % en volume d'un shampooing standard pendant trente secondes, puis rincée avec 200 ml d'eau. Ce procédé est répété six fois.

On observe que l'intensité de la couleur des mèches colorées avec les compositions contenant les colorants 1 et 2 conformes à l'invention est plus importante que l'intensité de la couleur des mèches colorées avec les compositions comparatives contenant les colorants 3 et 4 : les compositions tinctoriales selon la présente invention permettent d'obtenir des colorations plus résistantes aux shampooings.

## Revendications

1. Colorant naphtalimide cationique correspondant aux composés de formule générale (I) ainsi que les dimères des composés (I) correspondant aux formules générales (II), (III) et (IV) suivantes: 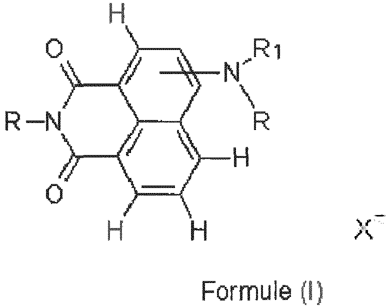 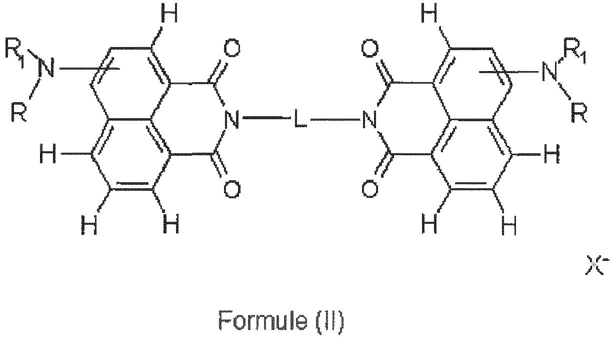 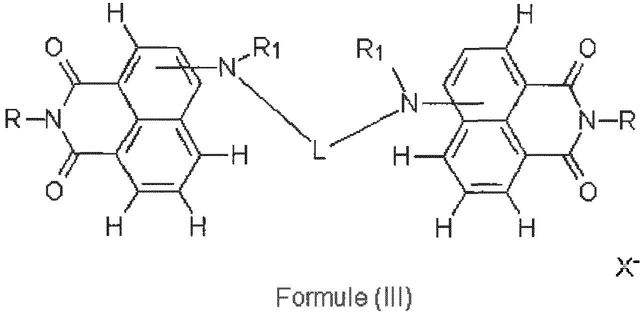 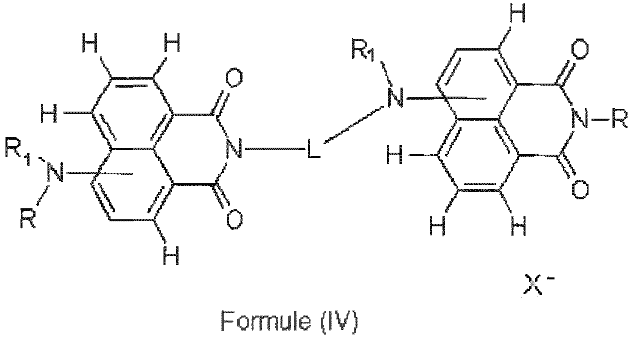 dans lesquelles :
- les radicaux R₁ pris séparément et indépendamment les uns des autres représentent un atome d'hydrogène ; un radical alkyle en C₁-C₄ éventuellement substitué par au moins un substituant choisi parmi les groupements :
• hydroxyle,
• alcoxy en C₁-C₄, (poly)hydroxyalcoxy en C₂-C₄,
• amino, amino substitué par un ou deux groupements alkyle identiques ou différents en C₁-C₄ éventuellement porteurs d'au moins un groupement hydroxyle, alcoxy en C₁-C₂, lesdits radicaux alkyle pouvant former avec l'atome d'azote auquel ils sont rattachés, un hétérocycle comprenant de 5 ou 7 chaînons, saturé ou insaturé, éventuellement aromatique, éventuellement substitué, comprenant éventuellement au moins un autre hétéroatome différent ou non de l'azote.
• Un radical alkylcarbonylamino (R'CO-NR-) dans lequel le radical R est un atome d'hydrogène, un radical alkyle en C₁-C₄ et le radical R' représente un radical alkyle en C₁-C₄, un radical phényle ;
• Un radical alkylsulfonyle (R-SO₂-) dans lequel le radical R représente un radical alkyle en C₁-C₄.
• Un radical alkylsulfinyle (R-SO-) dans lequel le radical R représente un radical alkyle en C₁-C₄.
• Un radical alkylcarbonyle (R-CO-) dans lequel le radical R représente un radical alkyle en C₁-C₄,
- les radicaux R pris séparément et indépendamment les uns des autres représentent un atome d'hydrogène ; un radical aryle ou arylalkyle dont la partie aryle est éventuellement substituée ; un radical alkyle en Ci-Cs étant éventuellement substitué par au moins un substituant choisi parmi les groupements :
• hydroxyle,
• alcoxy en C₁-C₄, (poly)hydroxyalcoxy en C₂-C₄,
• amino, amino substitué par un ou deux groupements alkyle identiques ou différents en C₁-C₄ éventuellement porteurs d'au moins un groupement hydroxyle, alcoxy en C₁-C₂, lesdits radicaux alkyle pouvant former avec l'atome d'azote auquel ils sont rattachés, un hétérocycle comprenant de 5 ou 7 chaînons, saturé ou insaturé, éventuellement aromatique, éventuellement substitué, comprenant éventuellement au moins un autre hétéroatome différent ou non de l'azote.
• Un radical alkylcarbonylamino (R'CO-NR-) dans lequel le radical R est un atome d'hydrogène, un radical alkyle en C₁-C₄ et le radical R' représente un radical alkyle en C₁-C₄, un radical phényle ;
• Un radical alkylsulfonyle (R-SO₂-) dans lequel le radical R représente un radical alkyle en C₁-C₄.
• Un radical alkylsulfinyle (R-SO-) dans lequel le radical R représente un radical alkyle en C₁-C₄.
• Un radical alkylcarbonyle (R-CO-) dans lequel le radical R représente un radical alkyle en C₁-C₄ ;
Ces radicaux R étant éventuellement interrompus par un ou plusieurs hétéroatomes choisis parmi l'oxygène, l'azote et le soufre, et
Ces radicaux R étant substitués par au moins un groupement correspondant aux formules (a) et (b) suivantes : 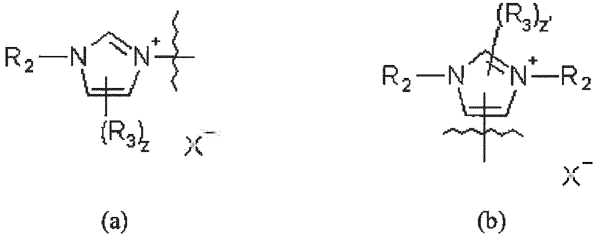 dans lesquelles le radical R₂ représente un radical alkyle en C₁-C₆ éventuellement substitué, le radical R₃ représente un atome d'halogène, un radical alkyle en C₁-C₆ éventuellement substitué, un radical monohydroxylalkyle en C₁-C₄, un radical alkoxy en C₁-C₄, un radical hydroxycarbonyl, un radical alkyl(C₁-C₆)thio, un radical amino disubstitué par un radical alkyle (C₁-C₄), z est un nombre entier compris entre 0 et 3 compris, z' est un nombre entier compris entre 0 et 2 compris,
- L est un bras de liaison cationique alkylène en C₂-C₂₀ interrompu par au moins un groupement correspondant aux formules suivantes : 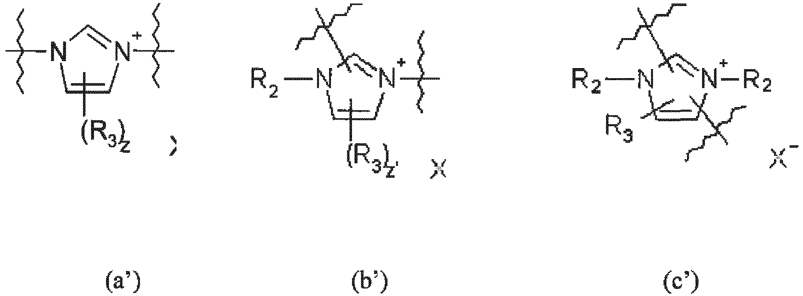 dans lesquelles le radical R₂ représente un radical alkyle en C₁-C₆ éventuellement substitué, le radical R₃ représente un atome d'halogène, un radical alkyle en C₁-C₆ éventuellement substitué, un radical monohydroxylalkyle en C₁-C₄, un radical alkoxy en C₁-C₄, un radical hydroxycarbonyl, un radical alkyl(C₁-C₆)thio, un radical amino disubstitué par un radical alkyle (C₁-C₄), z est un nombre entier compris entre 0 et 3 compris, z' est un nombre entier compris entre 0 et 2 compris,
• X⁻ représente un contre-ion permettant d'assurer l'électroneutralité des composés de formule (I) à (IV) ;
ainsi que leurs sels d'addition avec un acide et leurs solvates.

2. Colorant selon la revendication 1, dans lequel les radicaux R, identiques ou différents, indépendamment l'un de l'autre, représentent un atome d'hydrogène ; un radical alkyle en C₁-C₃, un radical hydroxyalkyle, un radical alcoxyalkyle, un radical alkyle en C₁-C₃ interrompu par au moins un groupement correspondant à la formule (a") suivante, un radical benzyle éventuellement substitué par un ou plusieurs radicaux, identiques ou différents, choisi parmi les groupements hydroxyle, alcoxyle tel que méthoxy, amino, (di)alkyl amino,

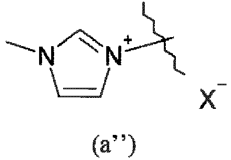

3. Colorant naphtalimide selon l'une quelconque des revendications précédentes choisi parmi :
- Le sel de 3-méthyl-1-{3-[(2-méthyl-1,3-dioxo-2,3-dihydro-1H-benzo[de]isoquinolin-6-yl)amino]propyl}-1H-imidazol-3-ium 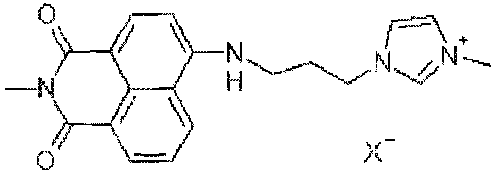
- Le sel de 1-{3-[6-[(2-hydroxyéthyl)amino]-1,3-dioxo-1H-benzo[de]isoquinolin-2(3H)-yl]propyl)-3-méthyl-1H-imidazol-3-ium 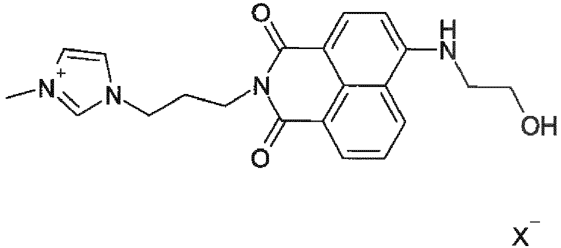
- Le sel de 3-méthyl-1-{3-[(6-{[3-(3-méthyl-1H-imidazol-3-ium-1-yl)propyl]ammo}-1,3 -dioxo-1H-benzo[de]isoquinolin-2(3H)-yl]propyl}-1H-imidazol-3 -ium 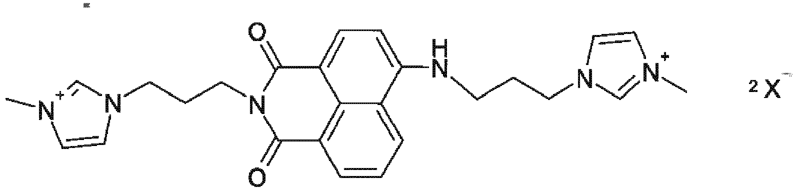
- Le sel de 3-{5-[(2-éthyl-1,3-dioxo-2,3-dihydro-1H-benzo[de]isoquinolin-6-yl)amino]pentyl}-1-{3-[(2-éthyl-1,3-dioxo-2,3-dihydro-1H-benzo[de]isoquinolin-6-yl)amino]propyl}-1H-imidazol-3-ium 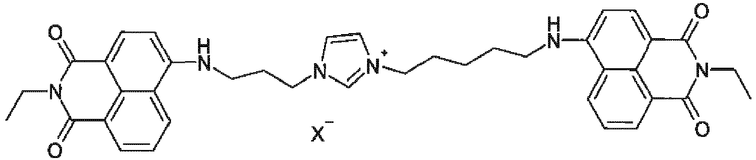
- Le sel de 3,3'-butane-l,4-diylbis(1-{5-[(2-éthyl-1,3-dioxo-2,3-dihydro-1H-benzo[de]isoquinolin-6-yl)amino]pentyl}-1H-imidazol-3-ium 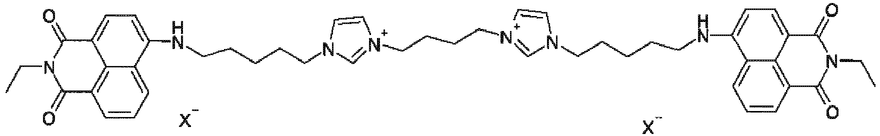
- Le sel de 3-{5-[(2-éthyl-1,3-dioxo-2,3-dihydro-1H-benzo[de]isoquinolin-6-yl)amino]pentyl}-1-[3-({2-[3-(1-méthyl-1H-imidazol-3-ium-3-yl)propyl]-1,3-dioxo-2,3-dihydro-1H-benzo [de]isoquinolin-6-yl} amino)propyl] -1H-imidazol-3 -ium 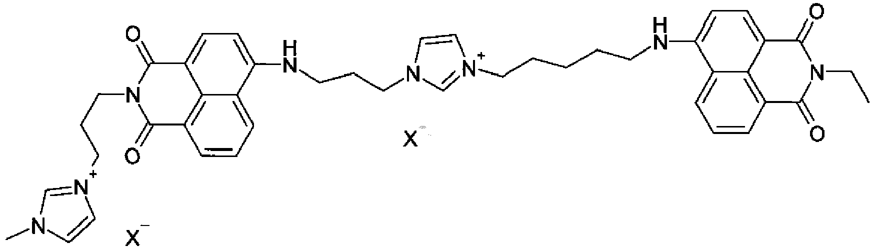
- Le sel de 1-{5-[(2-éthyl-1,3-dioxo-2,3-dihydro-1H-benzo[de]isoquinolin-6-yl)amino]pentyl}-3-(4-{1-[5-({2-[3-(1-methyl-1H-imidazol-3-ium-3-yl)propyl]-1,3-dioxo-2,3 -dihydro-1H-benzo[de]isoquinolin-6-yl}amino)pentyl]-1H-imidazol-3-ium-3-yl}butyl)-1H-imidazol-3 -ium 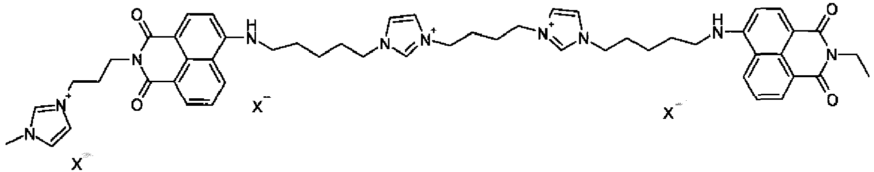
- Le sel de 1-methyl-3-{3-[6-[(3-{3-[5-({2-[3-(1-méthyl-1H-imidazol-3-ium-3-yl)propyl]-1,3-dioxo-2,3-dihydro-1H-benzo[de]isoquinolin-6-yl}amino)pentyl]-1H-imidazol-3-ium-1-yl}propyl)amino]-1,3-dioxo-1H-benzo[de]isoquinolin-2(3H)-yl]propyl}-1H-imidazol-3-ium 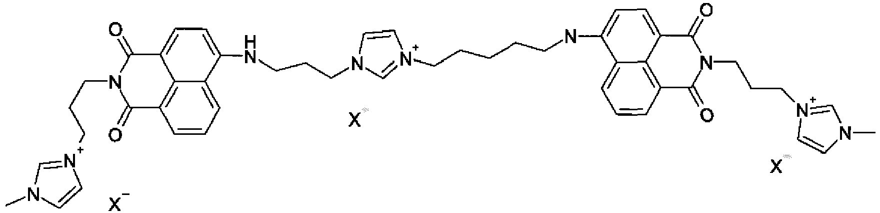
- Le sel de 3,3'-butane-1,4-diylbis{1-[5-({2-[3-(1-méthyl-1H-imidazol-3-ium-3-yl)propyl]-1,3-dioxo-2,3-dihydro-1H-benzo[de]isoquinolin-6-yl}amino)pentyl]-1H-imidazol-3-ium} 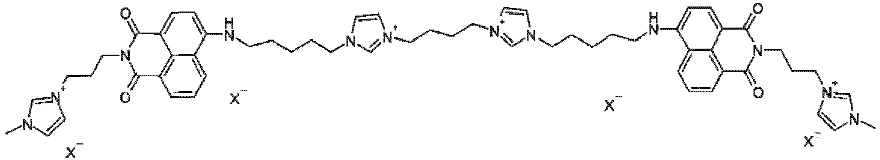
- Le sel de 1-méthyl-3-[3-({2-[3-(3-{5-[6-{[3-(1-méthyl-1H-imidazol-3-ium-3-yl)propyl]amino}-1,3-dioxo-1H-benzo[de]isoquinolin-2(3H)-yl]pentyl}-1H-imidazol-3-ium-1-yl)propyll-1,3-dioxo-2,3-dihydro-1H-benzo[de]isoquinolin-6-yl}amino)propyl]-1H-imidazol-3-ium 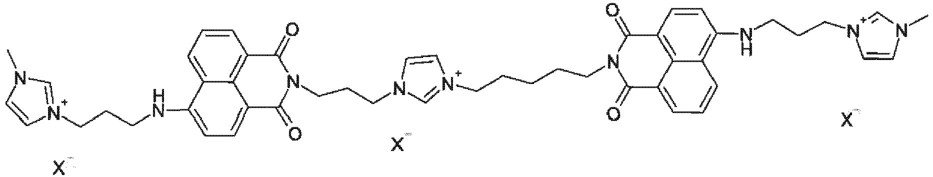
- Le sel de 3-{6-[6-[bis(2-hydroxyéthyl)amino]-1,3-dioxo-1H-benzo[de]isoqumolin-2(3H)-yl]hexyl}-1-{3-[(2-méthyl-1,3-dioxo-2,3-dihydro-1H-benzo[de]isoquinolin-6-yl)amino]propyl} -1H-imidazol-3-ium 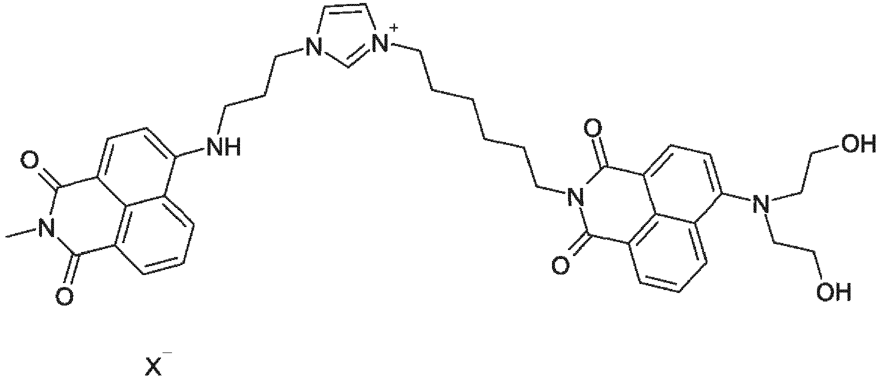
- Le sel de 3-méthyl-1-[3-({2-[6-(1-{3-[(2-méthyl-1,3-dioxo-2,3-dihydro-1H-benzo[de]isoquinolin-6-yl)amino]propyl}-1H-imidazol-3-ium-3-yl)hexyl]-1,3-dioxo-2,3-dihydro-1H-benzo[de]isoquinolin-6-yl}amino)propyl]-1H-imidazol-3-ium 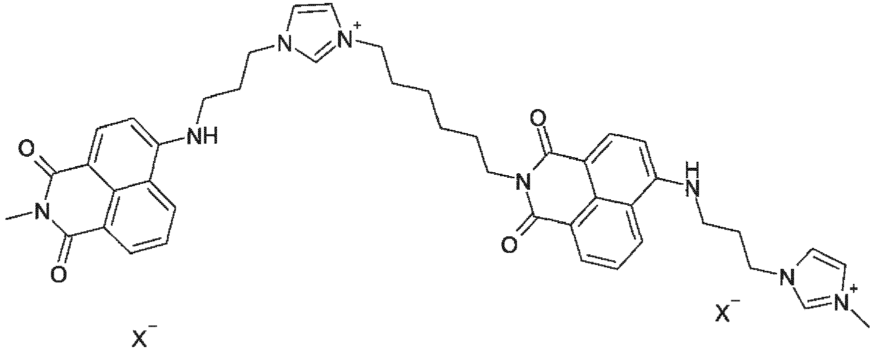
- Le sel de 1-méthyl-3-{3-[6-{[3-(3-{6-[6-{[3-(3-méthyl-1H-imidazol-3-ium-1-yl)propyl]amino}-1,3-dioxo-1H-benzo[de]isoqumolin-2(3H)-yl]hexyl}-1H-imidazol-3-ium-1-yl)propyl]amino}-1,3-dioxo-1H-benzo[de]isoquinolin-2(3H)-yl]propyl}-1H-imidazol-3-ium 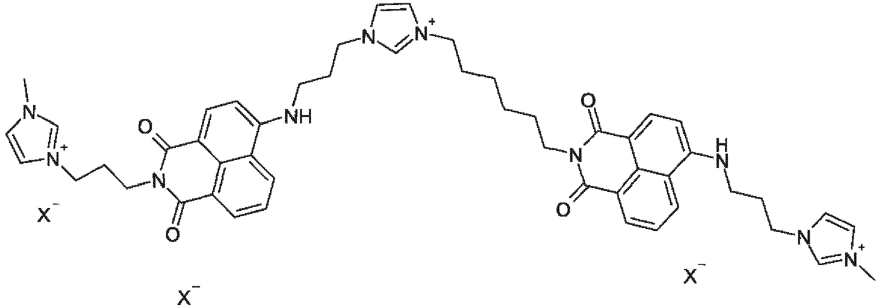
- Le sel de 3-méthyl-1-[3-({2-[6-(1-{6-[6-{[3-(1-métliyl-1H-imidazot-3-ium-3-yl)propyl]amino}-1,3-dioxo-1H-benzo[de]isoquinolin-2(3H)-yl]hexyl}-1H-imidazol-3-ium-3-yl)hexyl]-1,3-dioxo-2,3-dihydro-1H-benzo[de]isoquinolin-6-yl}amino)propyl]-1H-imidazol-3-ium

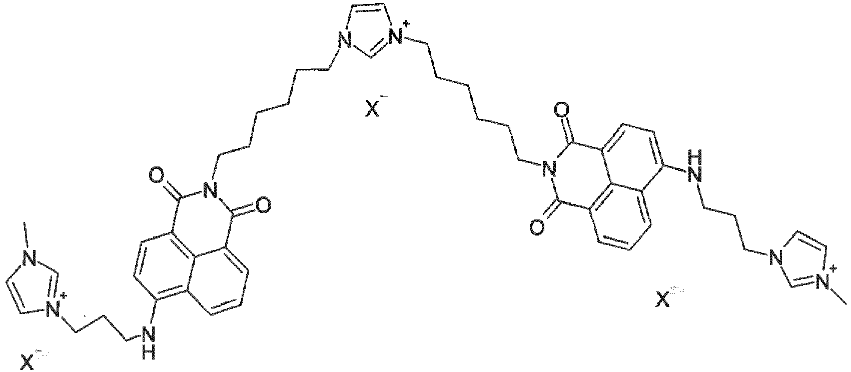

4. Colorant naphtalimide selon l'une quelconque des revendications précédentes de formule : 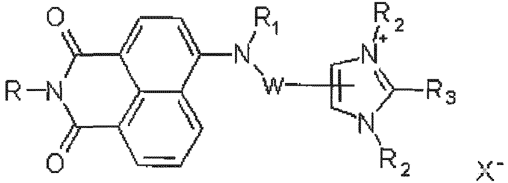 dans laquelle
- W représente un radical alkyle C₁-C₈, de préférence C₂-C₈ et de manière encore plus préférée en C₂-C₄,
- R, R₁, R₂, R₃ et X⁻ sont tels que définis à la revendication 1.

5. Colorant naphtalimide selon l'une quelconque des revendications 1 à 3 de formule : 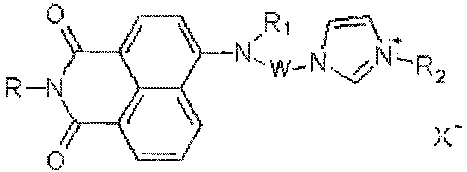 dans laquelle
- W représente un radical alkyle en C₁-C₈, de préférence C₂-C₈ et de manière encore plus préférée en C₂-C₄,
- R, R₁, R₂ et X⁻ sont tels que définis à la revendication 1.

6. Colorant naphtalimide selon l'une quelconque des revendications 1 à 3 de formule : 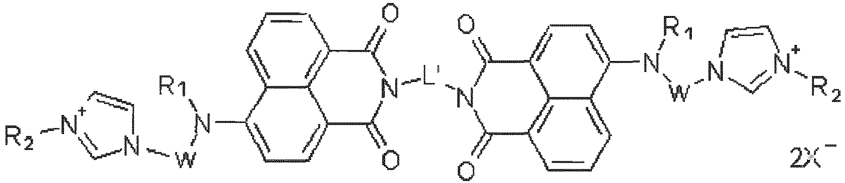 dans laquelle
- W représente un radical alkyle C₁-C₈, de préférence C₂-C₈ et de manière encore plus préférée en C₂-C₄,
- R₁, R₂ et X⁻ sont tels que définis à la revendication 1,
- L' est défini comme L.

7. Colorant naphtalimide selon l'une quelconque des revendications 1 à 3 de formule : 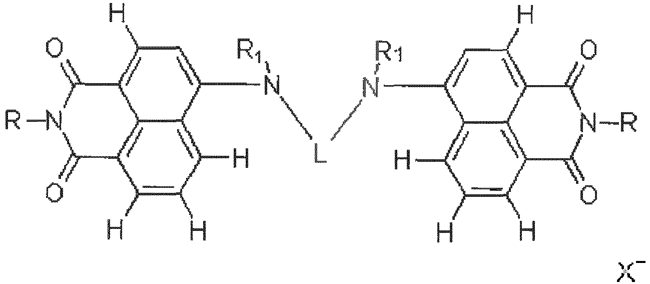 dans laquelle R, R₁, L et X⁻ sont tels que définis à la revendication 1.

8. Colorant naphtalimide selon l'une quelconque des revendications 1 à 3 de formule 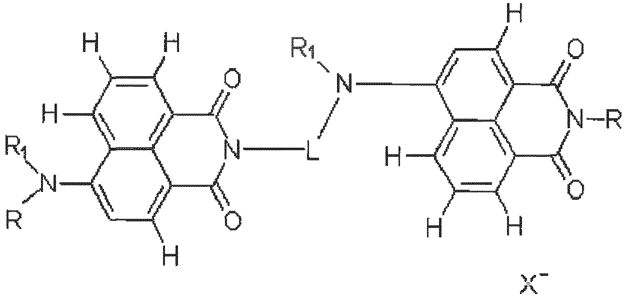 dans laquelle R, R₁, L et X⁻ sont tels que définis à la revendication 1.

9. Composition tinctoriale pour la teinture des fibres kératiniques comprenant dans un milieu de teinture approprié au moins un composé naphtalimide cationique selon l'une des revendications 1 à 8.

10. Composition selon la revendication 9 comprenant au moins un agent oxydant de type peroxyde et/ou un agent oxydant de type persel.

11. Procédé de coloration des fibres kératiniques comprenant l'application sur les fibres d'une composition telles que définie à la revendication 9 ou 10.

12. Utilisation de la composition selon l'une des revendications 9 ou 10 sur des fibres kératiniques présentant une hauteur de ton inférieure ou égale à 6, pour éclaircir lesdites fibres.

13. Kit de coloration des fibres kératiniques comprenant, d'une part, une composition comprenant au moins l'un des colorants naphtalimide des formules (I), (II), (III) ou (IV), leurs sels d'addition et/ou solvates, telle que définie à la revendication 9 ou 10, et d'autre part, un agent oxydant.

## Patentansprüche

1. Kationischer Naphthalimid-Farbstoff, der den Verbindungen der allgemeinen Formel (I) sowie den Dimeren der Verbindungen (I), die den folgenden Formeln (II), (III) und (IV) entsprechen, entspricht: 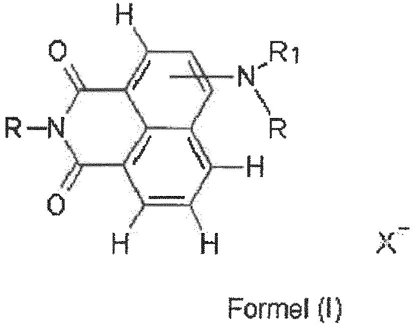 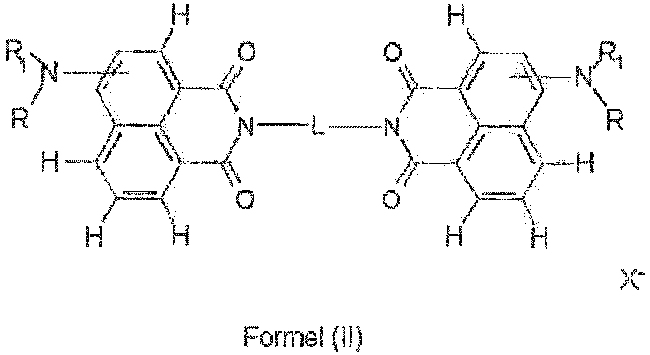 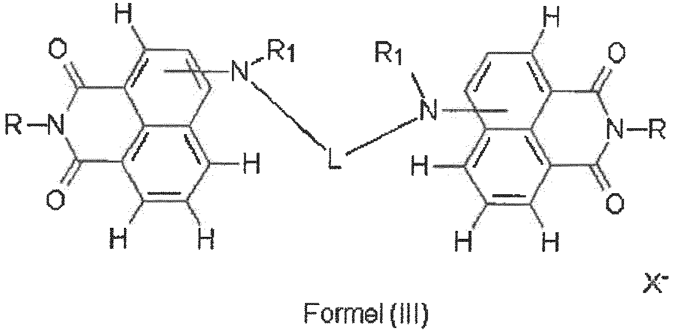 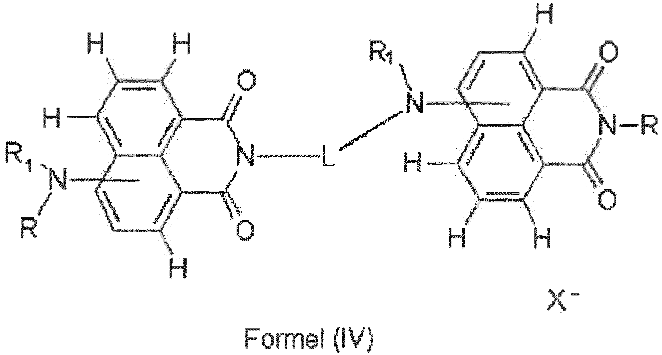 wobei:
die Reste R₁ separat und unabhängig voneinander für ein Wasserstoffatom oder einen C₁-C₄-Alkylrest, der gegebenenfalls durch mindestens einen Substituenten, der aus den Gruppen:
• Hydroxyl,
• C₁-C₄-Alkoxy, C₂-C₄-(Poly)hydroxyalkoxy,
• Amino, durch eine oder zwei gleiche oder verschiedene C₁-C₄-Alkylgruppen, die gegebenenfalls mindestens eine Hydroxyl- oder C₁-C₂-Alkoxygruppe tragen, substituiertes Amino, wobei die Alkylreste mit dem Stickstoffatom, an das sie gebunden sind, einen gesättigten oder ungesättigten, gegebenenfalls aromatischen, gegebenenfalls substituierten 5- oder 7-gliedrigen Heterocyclus bilden können, der gegebenenfalls mindestens ein weiteres Heteroatom, das von Stickstoff verschieden ist oder nicht, enthält,
• einem Alkylcarbonylaminorest (R'CO-NR-), wobei der Rest R für ein Wasserstoffatom oder einen C₁-C₄-Alkylrest steht und der Rest R' für einen C₁-C₄-Alkylrest oder einen Phenylrest steht,
• einem Alkylsulfonylrest (R-SO₂-), wobei der Rest R für einen C₁-C₄-Alkylrest steht,
• einem Alkylsulfinylrest (R-SO-), wobei der Rest R für einen C₁-C₄-Alkylrest steht,
• einem Alkylcarbonylrest (R-CO-), wobei der Rest R für einen C₁-C₄-Alkylrest steht,
ausgewählt ist, substituiert ist, stehen,
die Reste R separat und unabhängig voneinander für ein Wasserstoffatom, einen Aryl- oder Arylalkylrest, dessen Arylteil gegebenenfalls substituiert ist, oder einen C₁-C₈-Alkylrest, der gegebenenfalls durch mindestens einen Substituenten, der aus den Gruppen:
• Hydroxyl,
• C₁-C₄-Alkoxy, C₂-C₄-(Poly)hydroxyalkoxy,
• Amino, durch eine oder zwei gleiche oder verschiedene C₁-C₄-Alkylgruppen, die gegebenenfalls mindestens eine Hydroxyl- oder C₁-C₂-Alkoxygruppe tragen, substituiertes Amino, wobei die Alkylreste mit dem Stickstoffatom, an das sie gebunden sind, einen gesättigten oder ungesättigten, gegebenenfalls aromatischen, gegebenenfalls substituierten 5- oder 7-gliedrigen Heterocyclus bilden können, der gegebenenfalls mindestens ein weiteres Heteroatom, das von Stickstoff verschieden ist oder nicht, enthält,
• einem Alkylcarbonylaminorest (R'CO-NR-), wobei der Rest R für ein Wasserstoffatom oder einen C₁-C₄-Alkylrest steht und der Rest R' für einen C₁-C₄-Alkylrest oder einen Phenylrest steht,
• einem Alkylsulfonylrest (R-SO₂-), wobei der Rest R für einen C₁-C₄-Alkylrest steht,
• einem Alkylsulfinylrest (R-SO-), wobei der Rest R für einen C₁-C₄-Alkylrest steht,
• einem Alkylcarbonylrest (R-CO-), wobei der Rest R für einen C₁-C₄-Alkylrest steht,
ausgewählt ist, substituiert ist, stehen,
wobei diese Reste R gegebenenfalls durch ein oder mehrere Heteroatome, die aus Sauerstoff, Stickstoff und Schwefel ausgewählt sind, unterbrochen sind und
wobei diese Reste R durch mindestens eine Gruppe, die den folgenden Formeln (a) und (b) entspricht, substituiert sind: 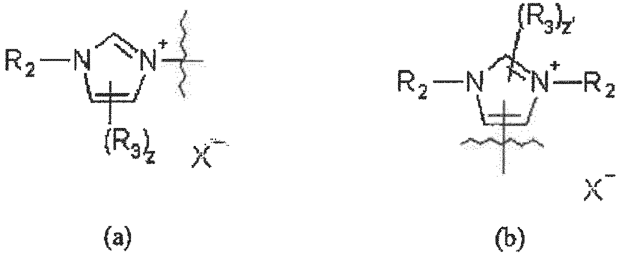 wobei der Rest R₂ für einen gegebenenfalls substituierten C₁-C₆-Alkylrest steht, der Rest R₃ für ein Halogenatom, einen gegebenenfalls substituierten C₁-C₆-Alkylrest, einen C₁-C₄-Mono-hydroxyalkylrest, einen C₁-C₄-Alkoxyrest, einen Hydroxycarbonylrest, einen (C₁-C₆)Alkylthiorest oder einen Aminorest, der zweifach durch einen (C₁-C₄)Alkylrest substituiert ist, steht, z für eine ganze Zahl zwischen 0 und 3 einschließlich steht und z' für eine ganze Zahl zwischen 0 und 2 einschließlich steht,
- L für einen kationischen C₂-C₂₀-Alkylen-Linkerarm steht, der durch mindestens eine Gruppe, die den folgenden Formeln entspricht, unterbrochen ist: 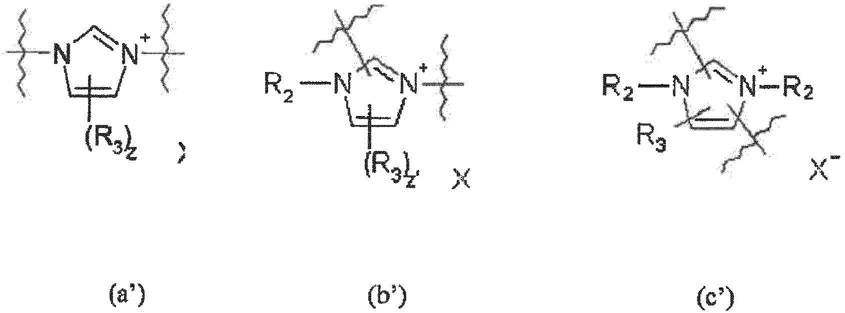 wobei der Rest R₂ für einen gegebenenfalls substituierten C₁-C₆-Alkylrest steht, der Rest R₃ für ein Halogenatom, einen gegebenenfalls substituierten C₁-C₆-Alkylrest, einen C₁-C₄-Mono-hydroxyalkylrest, einen C₁-C₄ -Alkoxyrest, einen Hydroxycarbonylrest, einen (C₁-C₆)Alkylthiorest oder einen Aminorest, der zweifach durch einen (C₁-C₄)Alkylrest substituiert ist, steht, z für eine ganze Zahl zwischen 0 und 3 einschließlich steht und z' für eine ganze Zahl zwischen 0 und 2 einschließlich steht,
- X⁻ für ein Gegenion, das die Elektroneutralität der Verbindungen der Formel (I) bis (IV) gewährleistet, steht;
sowie Additionssalze davon mit einer Säure und Solvate davon.

2. Farbstoff nach Anspruch 1, wobei die Reste R gleich oder verschieden sind und unabhängig voneinander für ein Wasserstoffatom, einen C₁-C₃-Alkylrest, einen Hydroxyalkylrest, einen Alkoxyalkylrest, einen C₁-C₃-Alkylrest, der durch mindestens eine Gruppe, die der folgenden Formel (a'') entspricht, unterbrochen ist, oder einen Benzylrest, der gegebenenfalls durch einen oder mehrere gleiche oder verschiedene Reste, die aus den Gruppen Hydroxyl, Alkoxy wie Methoxy, Amino und (Di)alkylamino ausgewählt sind, substituiert ist, stehen,

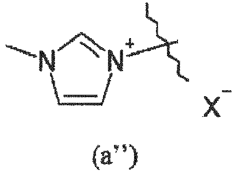

3. Naphtalimid-Farbstoff nach einem der vorhergehenden Ansprüche, ausgewählt aus:
- dem Salz von 3-Methyl-1-(3-[(2-methyl-1,3-dioxo-2,3-dihydro-1H-benzo[de]isochinolin-6-yl)amino]-propyl}-1H-imidazol-3-ium 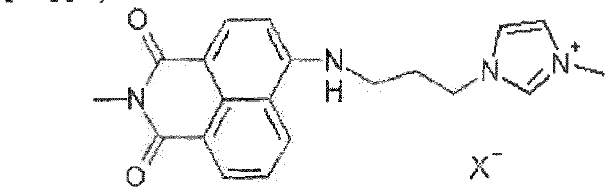
- dem Salz von 1-{3-[6-[(2-Hydroxyethyl)amino]-1,3-dioxo-1H-benzo[de]isochinolin-2(3H)-yl]-propyl}-3-methyl-1H-imidazol-3-ium 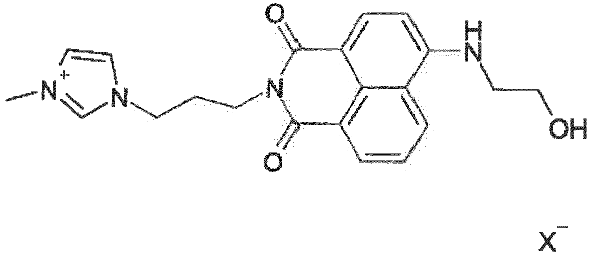
- dem Salz von 3-Methyl-1-{3-[(6-{[3-(3-methyl-1H-imidazol-3-ium-1-yl)propyl]amino}-1,3-dioxo-1H-benzo[de]isochinolin-2(3H)-yl]propyl}-1H-imidazol-3-ium 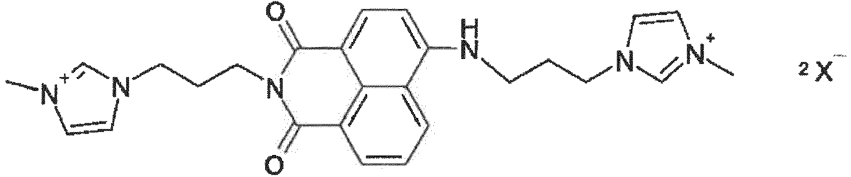
- dem Salz von 3-{5-[(2-Ethyl-1,3-dioxo-2,3-dihydro-1H-benzo[de]isochinolin-6-yl)amino]-pentyl}-1-{3-[(2-ethyl-1,3-dioxo-2,3-dihydro-1H-benzo[de]isochinolin-6-yl)amino]propyl}-1H-imidazol-3-ium 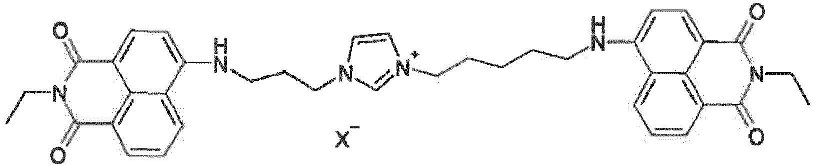
- dem Salz von 3,3'-Butan-1,4-diylbis(1-{5-[(2-ethyl-1,3-dioxo-2,3-dihydro-1H-benzo [de] isochinolin-6-yl)amino]pentyl}-1H-imidazol-3-ium 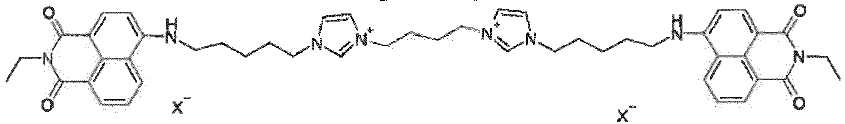
- dem Salz von 3-{5-[(2-Ethyl-1,3-dioxo-2,3-dihydro-1H-benzo[de]isochinolin-6-yl)amino]-pentyl}-1-[3-({2-[3-(1-methyl-1H-imidazol-3-ium-3-yl)propyl]-1,3-dioxo-2,3-dihydro-1H-benzo[de]-isochinolin-6-yl}amino)propyl]-1H-imidazol-3-ium 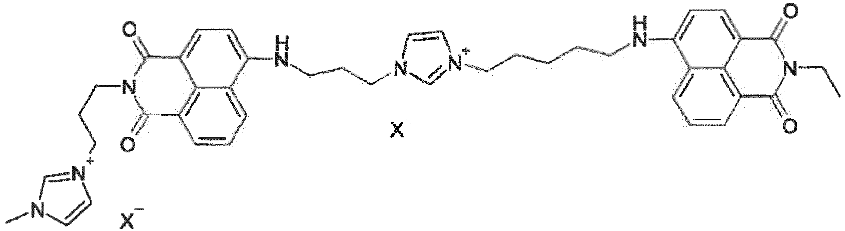
- dem Salz von 1-(5-[(2-Ethyl-1,3-dioxo-2,3-dihydro-1H-benzo[de]isochinolin-6-yl)amino]-pentyl}-3-(4-{1-[5-({2-[3-(1-methyl-1H-imidazol-3-ium-3-yl)propyl]-1,3-dioxo-2,3-dihydro-1H-benzo[de]isochinolin-6-yl}amino)pentyl]-1H-imidazol-3-ium-3-ylbutyl)-1H-imidazol-3-ium 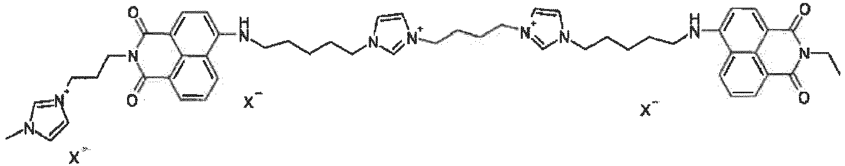
- dem Salz von 1-Methyl-3-{3-[6-[(3-{3-[5-({2-[3-(1-methyl-1H-imidazol-3-ium-3-yl)propyl]-1,3-dioxo-2,3-dihydro-1H-benzo[de]isochinolin-6-yl}amino)pentyl]-1H-imidazol-3-ium-1-yl}propyl)-aminol-1,3-dioxo-1H-benzo[delisochinolin-2(3H)-yl]propyl}-1H-imidazol-3-ium 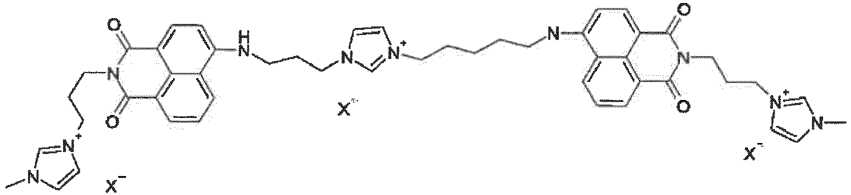
- dem Salz von 3,3'-Butan-1,4-diylbis{1-[5-({2-[3-(1-methyl-1H-imidazol-3-ium-3-yl)propyl]-1,3-dioxo-2,3-dihydro-1H-benzo[de]isochinolin-6-yl}amino)pentyl]-1H-imidazol-3-ium 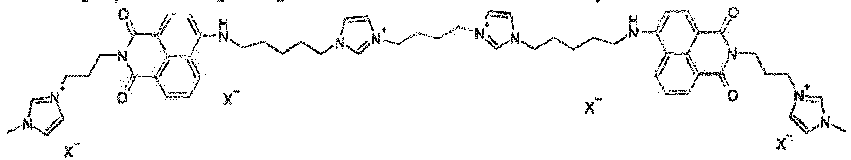
- dem Salz von 1-Methyl-3-[3-({2-[3-(3-{5-[6-{[3-(1-methyl-1H-imidazol-3-ium-3-yl)propyl]amino}-1,3-dioxo-1H-benzo[de]isochinolin-2(3H)-yl]pentyl}-1H-imidazol-3-ium-1-yl)propyl]-1,3-dioxo-2,3-dihydro-1H-benzo[de]isochinolin-6-yl)amino)propyl]-1H-imidazol-3-ium 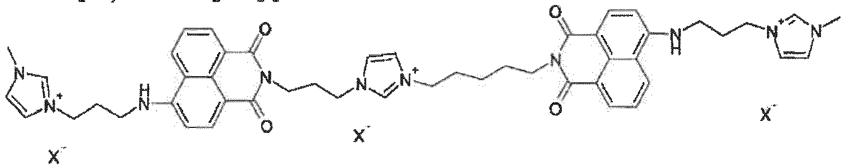
- dem Salz von 3-{6-[6-[Bis(2-hydroxyethyl)amino]-1,3-dioxo-1H-benzo[de]isochinolin-2(3H)-yl]hexyl}-1-{3-[(2-methyl-1,3-dioxo-2,3-dihydro-1H-benzo[de]isochinolin-6-yl)amino]propyl}-1H-imidazol-3-ium 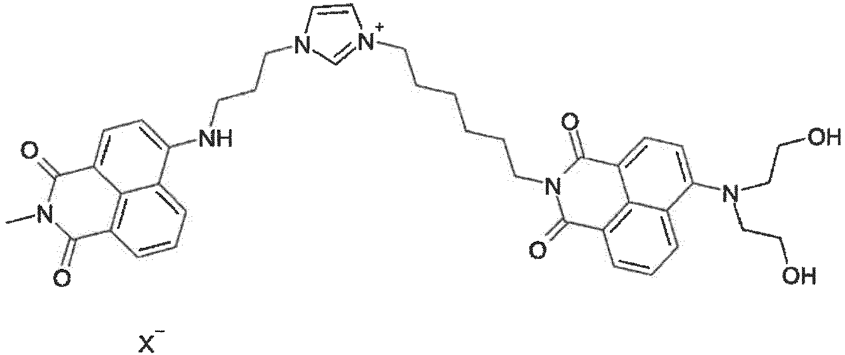
- dem Salz von 3-Methyl-1-[3-({2-[6-(1-{3-[(2-methyl-1,3-dioxo-2,3-dihydro-1H-benzo[de]isochinolin-6-yl)amino]propyl}-1H-imidazol-3-ium-3-yl)hexyl]-1,3-dioxo-2,3-dihydro-1H-benzo[de]isochinolin-6-yl}amino)propyl]-1H-imidazol-3-ium 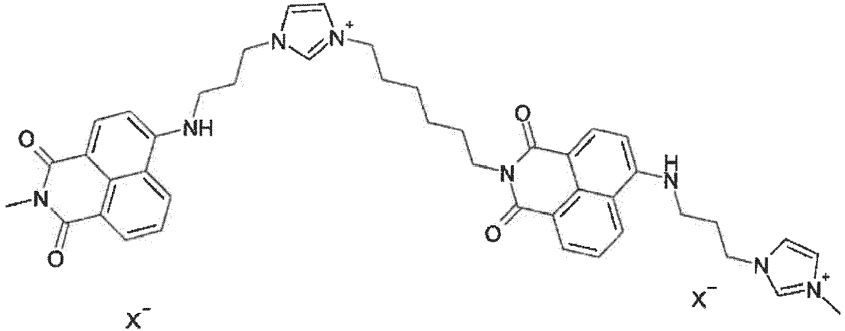
- dem Salz von 1-Methyl-3-{3-[6-{[3-(3-{6-[6-{[3-(3-methyl-1H-imidazol-3-ium-1-yl)propyl]amino}-1,3-dioxo-1H-benzo[de]isochinolin-2(3H)-yl]hexyl)-1H-imidazol-3-ium-1-yl)propyl]amino}-1,3-dioxo-1H-benzo[de]isochinolin-2(3H)-yl]propyl}-1H-imidazol-3-ium 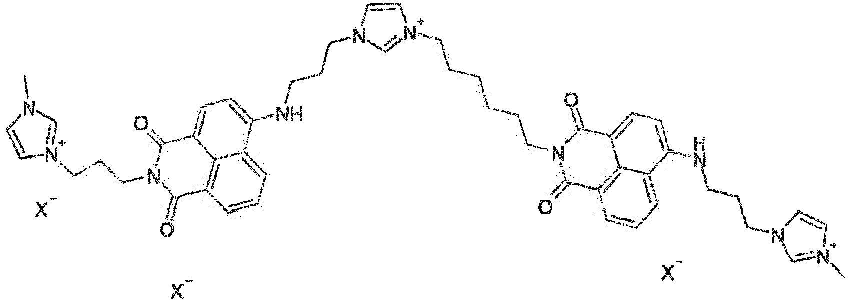
- dem Salz von 3-Methyl-1-[3-({2-[6-(1-{6-[6-{ [3-(1-methyl-1H-imidazol-3-ium-3-yl)propyl]amino}-1,3-dioxo-1H-benzo[de]isochinolin-2(3H)-yl]hexyl}-1H-imidazol-3-ium-3-yl)hexyl]-1,3-dioxo-2,3-dihydro-1H-benzo[de]isochinolin-6-yl)amino)-propyl] -1H-imidazol-3-ium

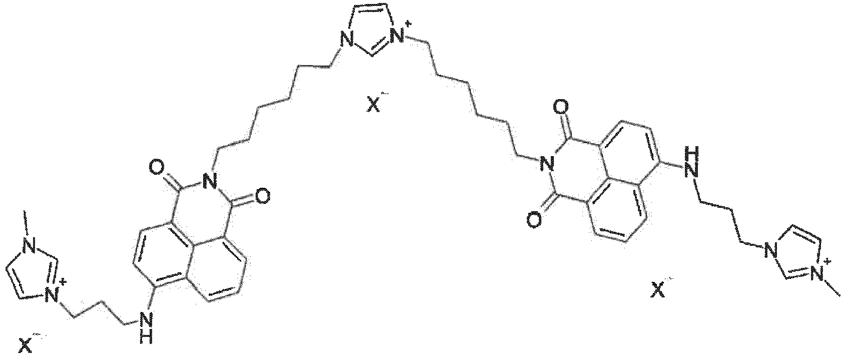

4. Naphthalimid-Farbstoff nach einem der vorhergehenden Ansprüche der Formel: 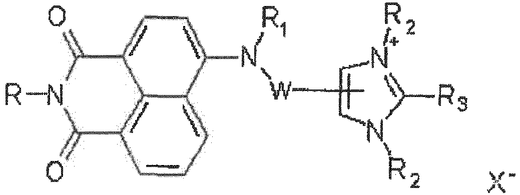 wobei
- W für einen C₁-C₈-, vorzugsweise C₂-C₈- und noch weiter bevorzugt C₂-C₄-Alkylrest steht,
- R, R₁, R₂, R₃ und X⁻ wie in Anspruch 1 definiert sind.

5. Naphthalimid-Farbstoff nach einem der Ansprüche 1 bis 3 der Formel: 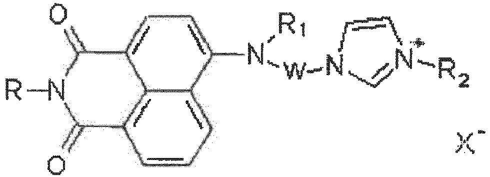 wobei
- W für einen C₁-C₈-, vorzugsweise C₂-C₈- und noch weiter bevorzugt C₂-C₄-Alkylrest steht,
- R, R₁, R₂ und X⁻ wie in Anspruch 1 definiert sind.

6. Naphthalimid-Farbstoff nach einem der Ansprüche 1 bis 3 der Formel: 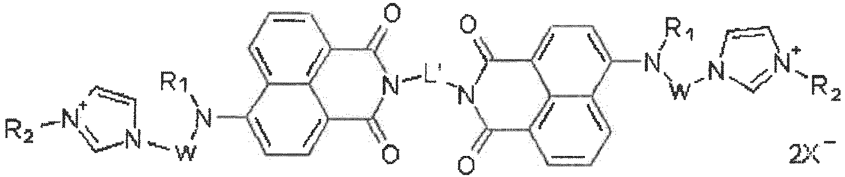 wobei
- W für einen C₁-C₈-, vorzugsweise C₂-C₈- und noch weiter bevorzugt C₂-C₄-Alkylrest steht,
- R₁, R₂ und X⁻ wie in Anspruch 1 definiert sind.

7. Naphthalimid-Farbstoff nach einem der Ansprüche 1 bis 3 der Formel: 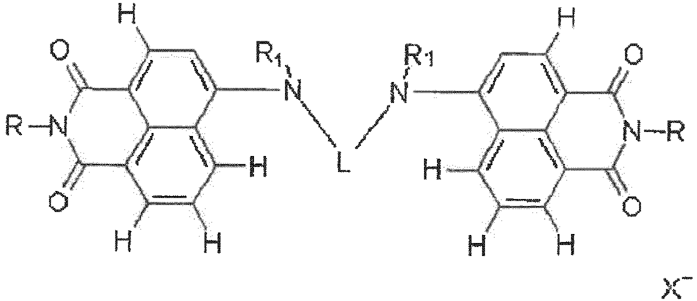 wobei R, R₁, L und X⁻ wie in Anspruch 1 definiert sind.

8. Naphthalimid-Farbstoff nach einem der Ansprüche 1 bis 3 der Formel: 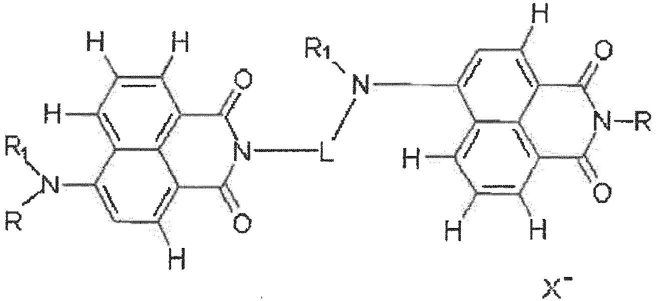 wobei R, R₁, L und X⁻ wie in Anspruch 1 definiert sind.

9. Färbezusammensetzung zum Färben von Keratinfasern, die in einem geeigneten Färbemedium mindestens eine kationische Naphthalimid-Verbindung nach einem der Ansprüche 1 bis 8 umfasst.

10. Zusammensetzung nach Anspruch 9, die mindestens ein Oxidationsmittel vom Wasserstoffperoxid-Typ und/oder ein Oxidationsmittel vom Persalz-Typ umfasst.

11. Verfahren zum Färben von Keratinfasern, bei dem man auf die Fasern eine Zusammensetzung gemäß Anspruch 9 oder 10 aufbringt.

12. Verwendung der Zusammensetzung nach einem der Ansprüche 9 oder 10 auf Keratinfasern mit einer Tonhöhe kleiner oder gleich 6 zum Aufhellen der Fasern.

13. Kit zum Färben von Keratinfasern, das einerseits eine Zusammensetzung, die mindestens einen der Naphthalimid-Farbstoffe der Formel (I), (II), (III) oder (IV), Additionssalze und/oder Solvate davon umfasst, gemäß Anspruch 9 oder 10 und andererseits ein Oxidationsmittel umfasst.

## Claims

1. Cationic naphthalimide dye corresponding to the compounds of general formula (I) and also the dimers of the compounds (I) corresponding to the following general formulae (II), (III) and (IV): 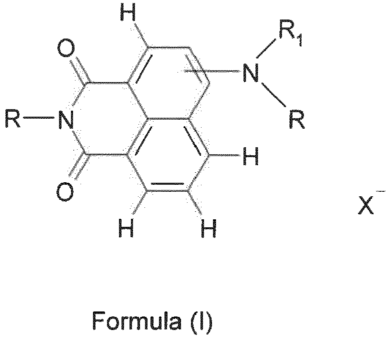 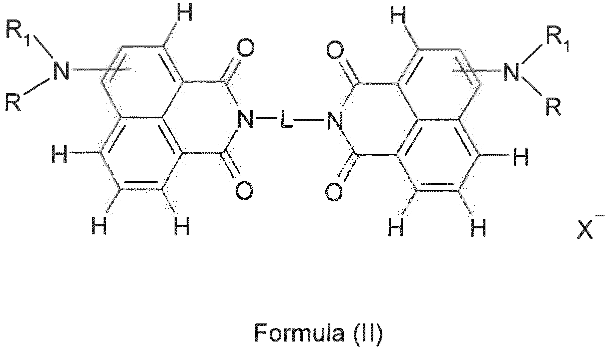 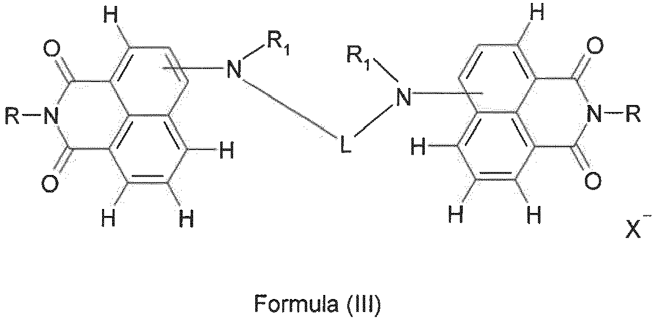 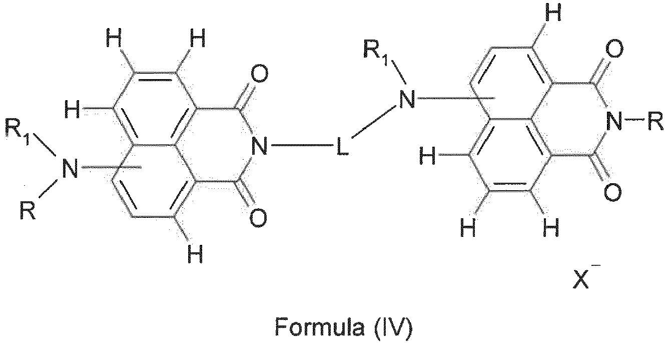 in which:
- the radicals R₁, taken separately and independently of one another, represent a hydrogen atom; a C₁-C₄ alkyl radical optionally substituted with at least one substituent chosen from the following groups:
• hydroxyl,
• C₁-C₄ alkoxy, C₂-C₄ (poly)hydroxyalkoxy,
• amino, amino substituted with one or two identical or different C₁-C₄ alkyl groups optionally carrying at least one hydroxyl or C₁-C₂ alkoxy group, it being possible for said alkyl radicals to form, with the nitrogen atom to which they are attached, a saturated or unsaturated, optionally aromatic, optionally substituted heterocycle comprising from 5 or 7 ring members, optionally comprising at least one other heteroatom which may or may not be different from nitrogen,
• an alkylcarbonylamino radical (R'CO-NR-) in which the radical R is a hydrogen atom, a C₁-C₄ alkyl radical, and the radical R' represents a C₁-C₄ alkyl radical, a phenyl radical;
• an alkylsulphonyl radical (R-SO₂-) in which the radical R represents a C₁-C₄ alkyl radical,
• an alkylsulphinyl radical (R-SO-) in which the radical R represents a C₁-C₄ alkyl radical,
• an alkylcarbonyl radical (R-CO-) in which the radical R represents a C₁-C₄ alkyl radical.
- the radicals R, taken separately and independently of one another, represent a hydrogen atom; an aryl or arylalkyl radical, in which the aryl part is optionally substituted; a C₁-C₈ alkyl radical which is optionally substituted with at least one substituent chosen from the following groups:
• hydroxyl,
• C₁-C₄ alkoxy, C₂-C₄ (poly) hydroxyalkoxy,
• amino, amino substituted with one or two identical or different C₁-C₄ alkyl groups optionally carrying at least one hydroxyl or C₁-C₂ alkoxy group, it being possible for said alkyl radicals to form, with the nitrogen atom to which they are attached, a saturated or unsaturated, optionally aromatic, optionally substituted heterocycle comprising from 5 or 7 ring members, optionally comprising at least one other heteroatom which may or may not be different from nitrogen,
• an alkylcarbonylamino radical (R'CO-NR-) in which the radical R is a hydrogen atom, a C₁-C₄ alkyl radical, and the radical R' represents a C₁-C₄ alkyl radical, a phenyl radical;
• an alkylsulphonyl radical (R-SO₂-) in which the radical R represents a C₁-C₄ alkyl radical,
• an alkylsulphinyl radical (R-SO-) in which the radical R represents a C₁-C₄ alkyl radical,
• an alkylcarbonyl radical (R-CO-) in which the radical R represents a C₁-C₄ alkyl radical;
these radicals R being optionally interrupted with one or more heteroatoms chosen from oxygen, nitrogen and sulfur, and
these radicals are being substituted with at least one group corresponding to the following formulae (a) and (b) : 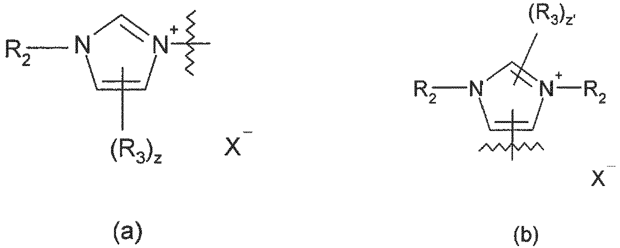 in which the radical R₂ represents an optionally substituted C₁-C₆ alkyl radical, the radical R₃ represents a halogen atom, an optionally substituted C₁-C₆ alkyl radical, a C₁-C₄ monohydroxyalkyl radical, a C₁-C₄ alkoxy radical, a hydroxycarbonyl radical, a (C₁-C₆)alkylthio radical, or an amino radical disubstituted with a (C₁-C₄) alkyl radical, z is an integer between 0 and 3 inclusive, z' is an integer between 0 and 2 inclusive,
- L is a C₂-C₂₀ alkylene cationic linker arm interrupted with at least one group corresponding to the following formulae : 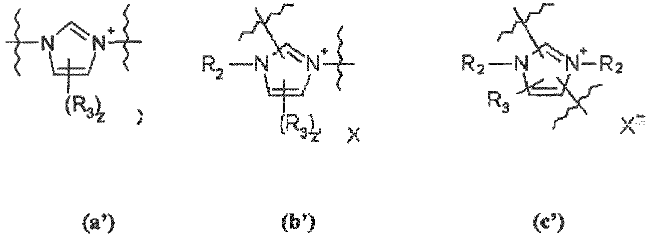 in which the radical R₂ represents an optionally substituted C₁-C₆ alkyl radical, the radical R₃ represents a halogen atom, an optionally substituted C₁-C₆ alkyl radical, a C₁-C₄ monohydroxyalkyl radical, a C₁-C₄ alkoxy radical, a hydroxycarbonyl radical, a (C₁-C₆)alkylthio radical, or an amino radical disubstituted with a (C₁-C₄) alkyl radical, z is an integer between 0 and 3 inclusive, z' is an integer between 0 and 2 inclusive,
• X⁻ represents a counterion for ensuring the electroneutrality of the compounds of formulae (I) to (IV); and also their addition salts with an acid and their solvates.

2. Dye according to Claim 1, in which the radicals R, which may be identical or different, independently of one another, represent a hydrogen atom; a C₁-C₃ alkyl radical, a hydroxyalkyl radical, an alkoxyalkyl radical, a C₁-C₃ alkyl radical interrupted with at least one group corresponding to the following formula (a"), a benzyl radical optionally substituted with one or more radicals, which may be identical or different, chosen from the groups hydroxyl, alkoxyl such as methoxy, amino and (di)alkylamino

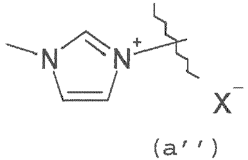

3. Naphthalimide dye according to any one of the preceding claims, chosen from:
- The 3-methyl-1-{3-[(2-methyl-1,3-dioxo-2,3-dihydro-1H-benzo[de]isoquinolin-6-yl)amino]propyl}-1H-imidazol-3-ium salt 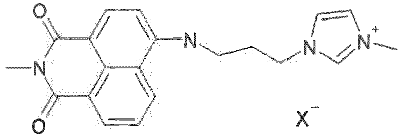
- The 1-{3-[6-[(2-hydroxyethyl)amino]-1,3-dioxo-1H-benzo[de]isoquinolin-2(3H)-yl]propyl}-3-methyl-1H-imidazol-3-ium salt 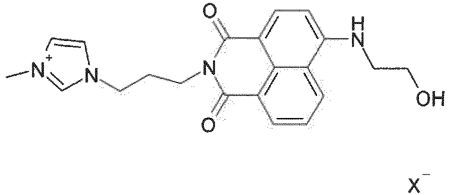
- The 3-methyl-1-{3-[(6-{[3-(3-methyl-1H-imidazol-3-ium-1-yl)propyl]amino}-1,3-dioxo-1H-benzo[de]isoquinolin-2(3H)-yl]propyl}-1H-imidazol-3-ium salt: 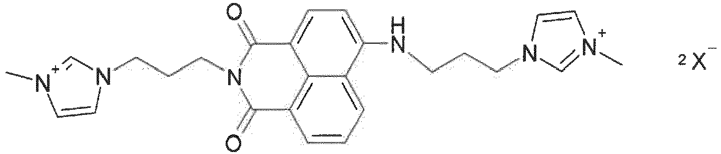
- The 3-{5-[(2-ethyl-1,3-dioxo-2,3-dihydro-1H-benzo[de]isoquinolin-6-yl)amino]pentyl}-1-{3-[(2-ethyl-1,3-dioxo-2,3-dihydro-1H-benzo[de]isoquinolin-6-yl)amino]propyl}-1H-imidazol-3-ium salt 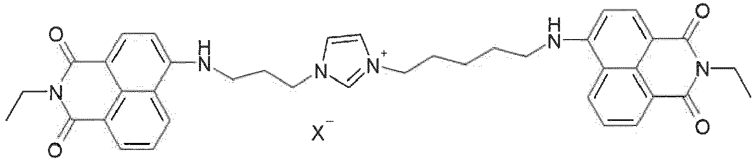
- The 3,3'-butane-1,4-diylbis(1-{5-[(2-ethyl-1,3-dioxo-2,3-dihydro-1H-benzo[de]isoquinolin-6-yl)amino]pentyl}-1H-imidazol-3-ium salt 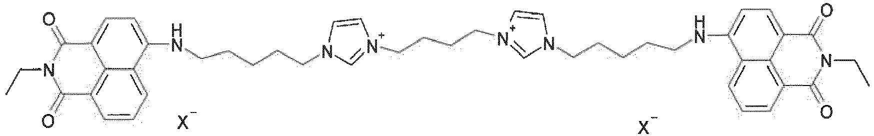
- The 3-{5-[(2-ethyl-1,3-dioxo-2,3-dihydro-1H-benzo[de]isoquinolin-6-yl}amino]pentyl}-1-[3-({2-[3-(1-methyl-1H-imidazol-3-ium-3-yl)propyl]-1,3-dioxo-2,3-dihydro-1H-benzo[de]isoquinolin-6-yl}amino)propyl]-1H-imidazol-3-ium salt 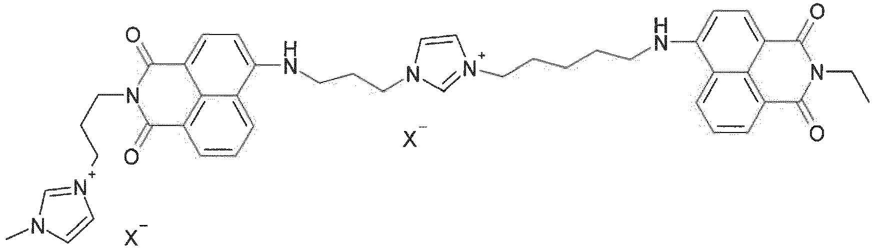
- The 1-{5-[(2-ethyl-1,3-dioxo-2,3-dihydro-1H-benzo[de]isoquinolin-6-yl)amino]pentyl}-3-(4-{1-[5-({2-[3-(1-methyl-1H-imidazol-3-ium-3-yl)propyl]-1,3-dioxo-2,3-dihydro-1H-benzo[de]isoquinolin-6-yl)amino)pentyl]-1H-imidazol-3-ium-3-yl}butyl)-1H-imidazol-3-ium salt 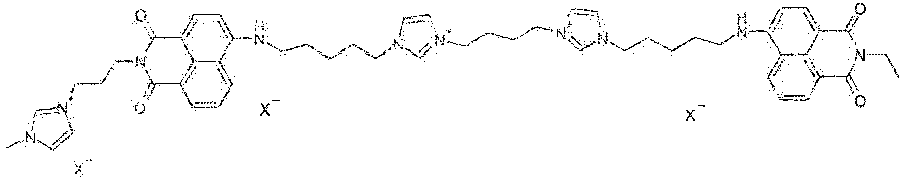
- The 1-methyl-3-{3-[6-[(3-{3-[5-({2-[3-(1-methyl-1H-imidazol-3-ium-3-yl)propyl]-1,3-dioxo-2,3-dihydro-1H-benzo[de]isoquinolin-6-yl}amino)pentyl]-1H-imidazol-3-ium-1-yl}propyl)amino]-1,3-dioxo-1H-benzo[de]isoquinolin-2(3H)-yl]propyl}-1H-imidazol-3-ium salt 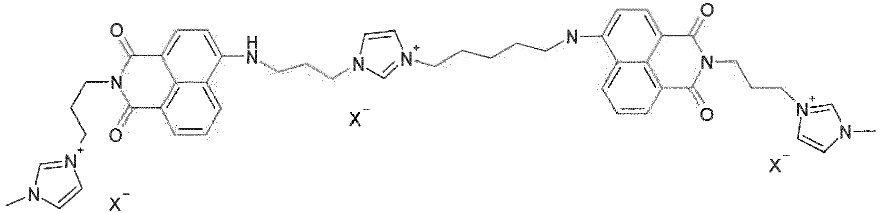
- The 3,3'-butane-1,4-diylbis{1-[5-({2-[3-(1-methyl-1H-imidazol-3-ium-3-yl)propyl]-1,3-dioxo-2,3-dihydro-1H-benzo[de]isoquinolin-6-yl}amino)pentyl]-1H-imidazol-3-ium} salt 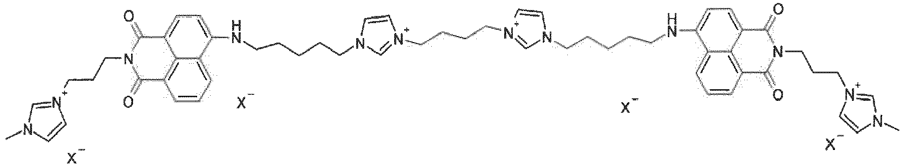
- The 1-methyl-3-[3-({2-[3-(3-{5-[6-{ [3-(1-methyl-1H-imidazol-3-ium-3-yl)propyl]amino}-1,3-dioxo-1H-benzo[de]isoquinolin-2(3H)-yl]pentyl}-1H-imidazol-3-ium-1-yl)propyl]-1,3-dioxo-2,3-dihydro-1H-benzo[de]isoquinolin-6-yl}amino)propyl]-1H-imidazol-3-ium salt 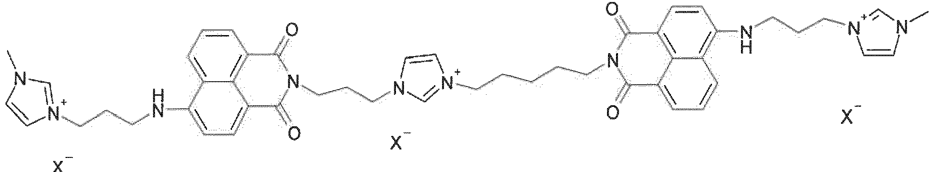
- The 3-{6-[6-[bis(2-hydroxyethyl)amino]-1,3-dioxo-1H-benzo[de]isoquinolin-2(3H)-yl]hexyl}-1-{3-[(2-methyl-1,3-dioxo-2,3-dihydro-1H-benzo[de]isoquinolin-6-yl)amino]propyl}-1H-imidazol-3-ium salt 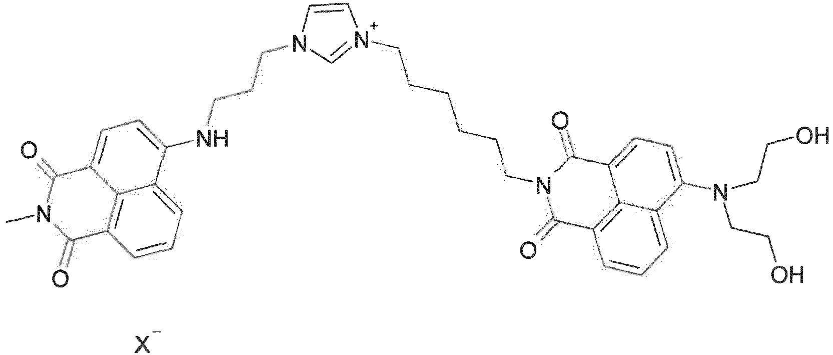
- The 3-methyl-1-[3-({2-[6-(1-{3-[(2-methyl-1,3-dioxo-2,3-dihydro-1H-benzo[de]isoquinolin-6-yl)amino]propyl}-1H-imidazol-3-ium-3-yl)hexyl]-1,3-dioxo-2,3-dihydro-1H-benzo[de]isoquinolin-6-yl}amino)propyl]-1H-imidazol-3-ium salt 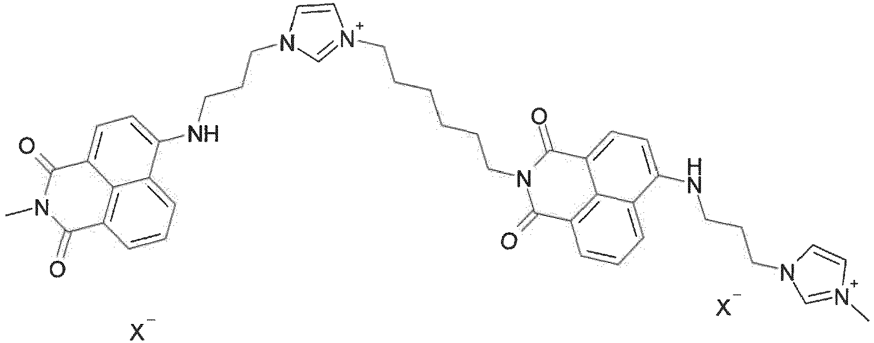
- The 1-methyl-3-{3-[6-{[3-(3-{6-[6-{[3-(3-methyl-1H-imidazol-3-ium-1-yl)propyl]amino}-1,3-dioxo-1H-benzo[de]isoquinolin-2(3H)-yl]hexyl}-1H-imidazol-3-ium-1-yl)propyl]amino)-1,3-dioxo-1H-benzo[de]isoquinolin-2(3H)-yl]propyl}-1H-imidazol-3-ium salt 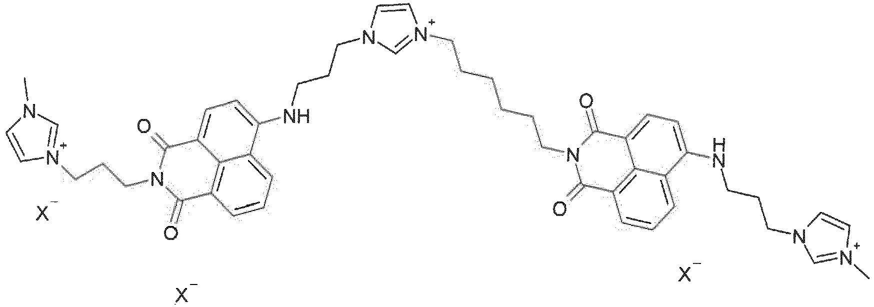
- The 3-methyl-1-[3-({2-[6-(1-{6-[6-{[3-(1-methyl-1H-imidazol-3-ium-3-yl)propyl]amino}-1,3-dioxo-1H-benzo[de]isoquinolin-2(3H)-yl]hexyl}-1H-imidazol-3-ium-3-yl)hexyl]-1,3-dioxo-2,3-dihydro-1H-benzo[de]isoquinolin-6-yl}amino)propyl]-1H-imidazol-3-ium salt

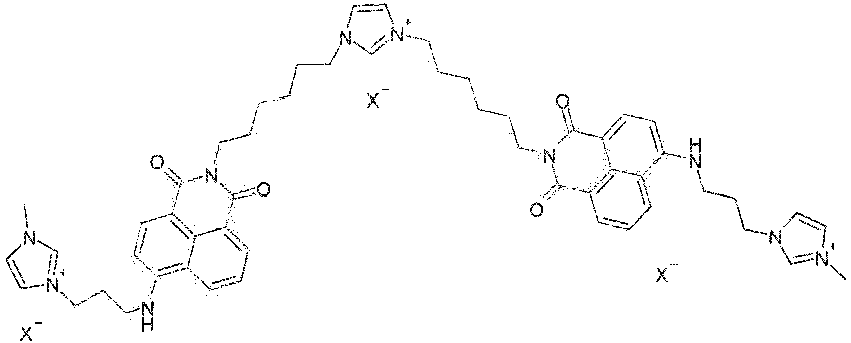

4. Naphthalimide dye according to any one of the preceding claims, of formula: 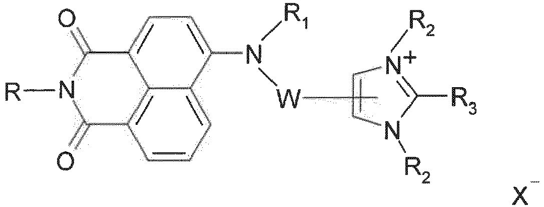 in which
- W represents a C₁-C₈ alkyl radical, preferably C₂-C₈, and even more preferably C₂-C₄,
- R, R₁, R₂, R₃ and X⁻ are as defined in Claim 1.

5. Naphthalimide dye according to any one of Claims 1 to 3, of formula: 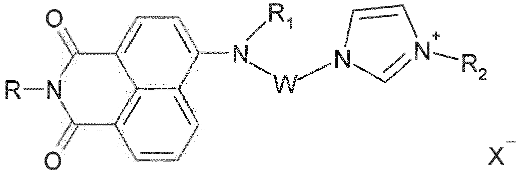 in which
- W represents a C₁-C₈ alkyl radical, preferably C₂-C₈, and even more preferably C₂-C₄,
- R, R₁, R₂ and X⁻ are as defined in Claim 1.

6. Naphthalimide dye according to any one of Claims 1 to 3, of formula: 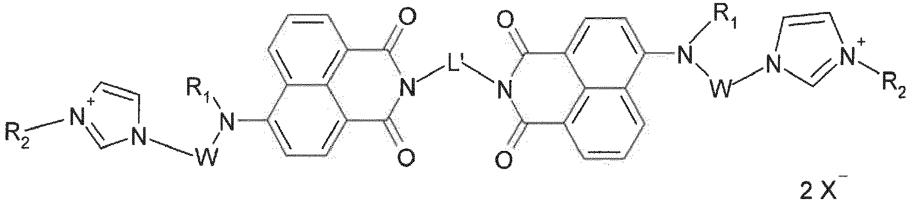 in which
- W represents a C₁-C₈ radical, preferably C₂-C₈, and even more preferably C₂-C₄,
- R₁, R₂ and X⁻ are as defined in Claim 1,
- L' is defined as L.

7. Naphthalimide dye according to any one of Claims 1 to 3, of formula: 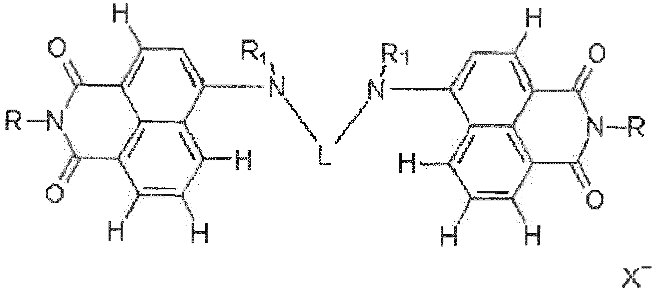 in which R, R₁, L and X⁻ are as defined in Claim 1.

8. Naphthalimide dye according to any one of Claims 1 to 3, of formula: 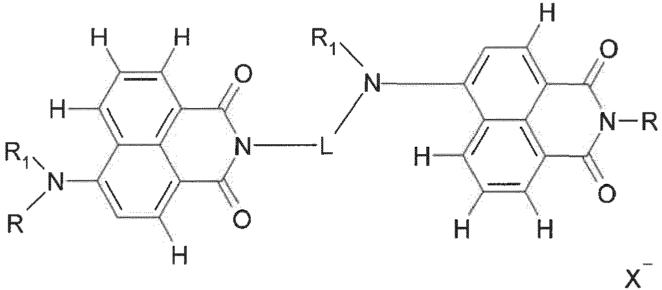 in which R, R₁, L and X⁻ are as defined in Claim 1.

9. Dye composition for the dyeing of keratin fibres, comprising, in a suitable dye medium, at least one cationic naphthalimide compound according to one of Claims 1 to 8.

10. Composition according to Claim 9, comprising at least one oxidizing agent of peroxide type and/or an oxidizing agent of persalt type.

11. Process for dyeing keratin fibres, comprising the application to the fibres of a composition as defined in Claim 9 or 10.

12. Use of the composition according to one of Claims 9 or 10 on keratin fibres having a tone depth of less than or equal to 6 in order to lighten said fibres.

13. Kit for dyeing keratin fibres, comprising, firstly, a composition comprising at least one of the naphthalimide dyes of formulae (I), (II), (III) or (IV), addition salts and/or solvates, as defined in Claims 9 or 10, and, secondly, an oxidizing agent.
